(19)  Europäisches Patentamt
European Patent Office
Office européen des brevets

(11)  **EP 4 204 144 B1**

(12)  ## EUROPEAN PATENT SPECIFICATION

(45) Date of publication and mention
of the grant of the patent:
**18.03.2026  Bulletin 2026/12**

(21) Application number: **21763400.5**

(22) Date of filing: **23.08.2021**

(51) International Patent Classification (IPC):
*B01J 23/52* (2006.01)   *B22F 1/00* (2022.01)
*B22F 9/24* (2006.01)   *B82Y 5/00* (2011.01)
*H01B 1/22* (2006.01)   *H05K 1/09* (2006.01)
*A61K 47/69* (2017.01)   *B01J 35/23* (2024.01)
*B22F 1/054* (2022.01)   *B22F 1/102* (2022.01)
*B01J 35/45* (2024.01)

(52) Cooperative Patent Classification (CPC):
(C-Sets available)
**B22F 1/054; B01J 23/52; B01J 35/45; B22F 1/102;**
**B22F 9/24; H01B 1/22;** B22F 1/0545; B22F 1/16;
B22F 2999/00; B82Y 5/00            (Cont.)

(86) International application number:
**PCT/GB2021/052190**

(87) International publication number:
**WO 2022/043671 (03.03.2022 Gazette 2022/09)**

(54) **METHOD OF PRODUCING A METAL NANOPARTICLE**

VERFAHREN ZUR HERSTELLUNG EINES METALLNANOPARTIKELS

MÉTHODE DE PRODUCTION D'UNE NANOPARTICULE MÉTALLIQUE

(84) Designated Contracting States:
**AL AT BE BG CH CY CZ DE DK EE ES FI FR GR
HR HU IE IS IT LI LT LU LV MC MK MT NL NO PL
PT RO RS SE SI SK SM TR**
Designated Validation States:
**KH MA TN**

(30) Priority: **25.08.2020  GB 202013271**

(43) Date of publication of application:
**05.07.2023  Bulletin 2023/27**

(73) Proprietor: **Metalchemy Limited**
**London**
**WC2H 9JQ (GB)**

(72) Inventor: **TROTTA, Federico**
**London WC2H 9JQ (GB)**

(74) Representative: **D Young & Co LLP**
**3 Noble Street**
**London EC2V 7BQ (GB)**

(56) References cited:
• **SOSNOWSKA MALWINA ET AL: "Green
synthesis of silver nanoparticles by using
aqueous mint (Mentha piperita) and cabbage
(Brassica oleracea var. capitata) extracts and
their antibacterial activity", ANNALS OF
WARSAW UNIVERSITY OF LIFE SCIENCES -
SGGW - ANIMAL SCIENCE, vol. 56, no. 1, 30 June
2017 (2017-06-30), pages 137 - 145,
XP055856096, ISSN: 1898-8830, Retrieved from
the Internet <URL:http://annals-wuls.sggw.pl/
files/files/animal/asc2017no561p137-145.pdf>
DOI: 10.22630/AAS.2017.56.1.16**
• **JASMINE JACOB ET AL: "A simple approach for
facile synthesis of Ag, anisotropic Au and
bimetallic (Ag/Au) nanoparticles using
cruciferous vegetable extracts", MATERIALS
SCIENCE AND ENGINEERING C, ELSEVIER
SCIENCE S.A, CH, vol. 32, no. 7, 30 April 2012
(2012-04-30), pages 1827 - 1834, XP028503967,
ISSN: 0928-4931, [retrieved on 20120509], DOI:
10.1016/J.MSEC.2012.04.072**

EP 4 204 144 B1

**(Cont. next page)**

- DEMIRBAS AYSE ET AL: "Biosynthesis of red cabbage extract directed Ag NPs and their effect on the loss of antioxidant activity", MATERIALS LETTERS, ELSEVIER, AMSTERDAM, NL, vol. 179, 10 May 2016 (2016-05-10), pages 20 - 23, XP029562459, ISSN: 0167-577X, DOI: 10.1016/J.MATLET.2016.05.056
- HASSANI SADI MOHADESEH ET AL: "Plants used in folkloric medicine of Iran are exquisite bio-resources in production of silver nanoparticles", IET NANOBIOTECHNOLOGY, THE INSTITUTION OF ENGINEERING AND TECHNOLOGY, MICHAEL FARADAY HOUSE, SIX HILLS WAY, STEVENAGE, HERTS. SG1 2AY, UK, vol. 11, no. 3, 1 April 2017 (2017-04-01), pages 300 - 309, XP006060757, ISSN: 1751-8741, DOI: 10.1049/IET-NBT.2016.0114
- WIOLETTA FLORKIEWICZ ET AL: "Assessment of cytotoxicity and immune compatibility of phytochemicals-mediated biosynthesised silver nanoparticles using Cynara scolymus", IET NANOBIOTECHNOLOGY, THE INSTITUTION OF ENGINEERING AND TECHNOLOGY, MICHAEL FARADAY HOUSE, SIX HILLS WAY, STEVENAGE, HERTS. SG1 2AY, UK, vol. 13, no. 7, 8 July 2019 (2019-07-08), pages 726 - 735, XP006109171, ISSN: 1751-8741, DOI: 10.1049/IET-NBT.2018.5357
- AZIZIAN-SHERMEH OMID ET AL: "Facile, Low-Cost and Rapid Phytosynthesis of Stable and Eco-friendly Silver Nanoparticles Using(Choisy) and Study of Their Antimicrobial Activities", JOURNAL OF INORGANIC AND ORGANOMETALLIC POLYMERS AND MATERIALS, vol. 31, no. 1, 5 August 2020 (2020-08-05), pages 279 - 291, XP037338095, ISSN: 1574-1443, DOI: 10.1007/S10904-020-01691-7
- POURHASSAN-MOGHADDAM MOHAMMAD ET AL: "Watercress-based gold nanoparticles: biosynthesis, mechanism of formation and study of their biocompatibility in vitro", MICRO & NANO LETTERS, THE INSTITUTION OF ENGINEERING AND TECHNOLOGY, MICHAEL FARADAY HOUSE, SIX HILLS WAY, STEVENAGE, HERTS. SG1 2AY, UK, vol. 9, no. 5, 1 May 2014 (2014-05-01), pages 345 - 350, XP006048507, DOI: 10.1049/MNL.2014.0063
- JEONG SU J ET AL: "Nanoencapsulation of synergistic antioxidant fruit and vegetable concentrates and their stability during in vitro digestion", JOURNAL OF THE SCIENCE OF FOOD AND AGRICULTURE, vol. 100, no. 3, 21 November 2019 (2019-11-21), GB, pages 1056 - 1063, XP055856414, ISSN: 0022-5142, Retrieved from the Internet <URL:https://onlinelibrary.wiley.com/doi/full-xml/10.1002/jsfa.10110> DOI: 10.1002/jsfa.10110

(52) Cooperative Patent Classification (CPC): (Cont.)

C-Sets
B22F 2999/00, B22F 1/054, C22C 1/0466

**Description**

**Field of the Invention**

This invention relates to a method for producing a metal nanoparticle using a composition.

**Background to the Invention**

Metallic nanoparticles are typically defined as being particles of diameter under 100nm. These have a wide range of commercial applications, including in pharmaceuticals and electronics.

Various processes have been developed in the last 20 years in order to produce metal nanoparticles including both chemical and physical manufacturing routes. However, some of these methods give rise to environmental and safety concerns. It would be desirable to manufacture these products in a more environmentally friendly manner.

David L. Johnson et al., Micro & Nano Letters, 2020, 15(2), 110-113, describes the biosynthesis of silver nanoparticles using upland cress, their purification and characterisation. This process only uses upland cress as the source of antioxidants. The ratio of antioxidants used in the process, and the bioreactors used differ from that of the present invention. In particular, the reaction is less efficient in that it takes 6 hours at 37°C. Moreover, a different washing solution is used for centrifugation and the separation techniques are different. In particular, this process uses $AgNO_3$ as silver salt - leaching of nitrates into water supplies is known to be environmentally unfavourable.

Mohammad Pourhassan-Moghaddam et al., Micro & Nano Letters, 2014, 9(5), 345-350, describes watercress-based gold nanoparticles, their biosynthesis, mechanism of formation and their biocompatibility *in vitro.* This process uses $HAuCl_4.3H_2O$ to produce gold nanoparticles, and only uses watercress as the source of antioxidants. The bioreactor used differs from and is less efficient than that of the present invention.

US2012/0055873 describes the green synthesis of nanometals using plant extracts. This process uses commercially available plant extracts, such as green tea/coffee, to produce nanoparticles. The principal antioxidants used are polyphenols and caffeine, and the bioreactor also differs from that of the present invention. Moreover, this process also uses $AgNO_3$ as the source of silver ions.

US2011/0110723 describes green synthesis of nanometals using fruit extracts. This process uses fruits (which are rich in sugars) as the source of antioxidants, and the bioreactor also differs from that of the present invention. Moreover, this process also uses $AgNO_3$ as the source of silver ions.

Sosnowska Malwina et al, Annals of Warsaw University of Life Sciences - SGGW - Animal Science, 2017, 56(1), 137-145 describes the synthesis of silver nanoparticles using aqueous mint (*Mentha piperita*) and cabbage (*Brassica oleracea var. capitata*) extracts.

Jasmine Jacob et al, Materials Science and Engineering C, 2012, 32(7), 1827-1834 describes an approach for the synthesis of Ag, Au and bimetallic (Ag/Au) nanoparticles using cruciferous vegetable (green/red) extracts.

Demirbas Ayse et al, Materials Letters, 2016, 179, 20-23 describes the synthesis of gold nanoparticles using red cabbage extracts.

Hassani Sadi Mohadeseh et al, IET Nanobiotechnology, The Institution of Engineering and Technology, 2017, 11(3), 300-309 describes the use of Iranian medicinal plant species belonging to 49 families for the biosynthesis of colloidal silver nanoparticles (AgNPs).

Wioletta Florkiewicz et al, IET Nanobiotechnology, The Institution of Engineering and Technology, 2019, 13(7), 726-735 describes the phytochemicals-mediated biosynthesis of silver nanoparticles using leaf extracts and infusions from *Cynara scolymus.*

Azizian-shermeh Omid et al, Journal of Inorganic and Organometallic Polymers and Materials, 2021, 31(1), 279-291 describes the biosynthesis of silver nanoparticles (SNPs) using *Boerhavia elegans* (choisy).

Jeong Su J et al, Journal of the Science of Food and Agriculture, 2019, 100(3), 1056-1063 describes the encapsulation of selected fruit and vegetable concentrates in Chitosan/carrageenan (CSCR) nanoparticles.

**Summary of the Invention**

The present invention provides a method of producing a metal nanoparticle having a diameter between 1nm and 1μm, the method comprising:

    (a) providing a dissolved metal ion; and
    (b) contacting the dissolved metal ion with a composition comprising extracts from:

        (a) kale *(Brassica oleracea* Acephala group) and oregano *(Origanum vulgare);* and
        (b) at least one other plant selected from the group consisting of:

(i) artichoke (Cynara cardunculus);
(ii) red cabbage (Brassica oleracea var. Capitata group var. rubra);
(iii) rosemary (Salvia rosmarinus);
(iv) sage (Salvia officinalis);
(v) watercress (Nasturtium officinale);

such that the dissolved metal ion is reduced to form the metal nanoparticle.

**Brief Description of the Drawings**

Figure 1 is a process diagram showing a preferred method of producing the composition and the metal nanoparticle;
Figure 1A is a process diagram showing an alternative preferred method of producing the composition and the metal nanoparticle;
Figure 2 is a Pareto Frontier analysis showing sensitivity analysis on different constraint configurations for a number of compositions;
Figure 3 show the results of Pareto Optimal Frontier analysis (antioxidant composition vs blend cost per 100g) for nine blend compositions;
Figure 4 show the results of Pareto Optimal Frontier analysis of relative antioxidant activity relative to watercress for nine blend compositions;
Figure 5 shows the constituents of nine blend compositions;
Figure 6 shows the constituents of five preferred blend compositions which were DPPH-tested in Example 2;
Figure 7 shows the inhibition of DPPH by the preferred compositions as a function of antioxidant concentrations / Watercress and Curly Kale Concentrations;
Figure 8 shows the inhibition of DPPH as a function of antioxidant concentrations blend for five preferred blend compositions as tested in Example 2; Figure 9 shows the inhibition of DPPH for ascorbic acid as a function of concentration;
Figure 10 shows a linear regression of inhibition of DPPH for ascorbic acid as a function of concentration;
Figure 11 shows a linear regression of inhibition of DPPH for watercress as a function of concentration;
Figure 12 shows a linear regression of inhibition of DPPH for curly kale as a function of concentration;
Figure 13 shows a linear regression of inhibition of DPPH for the composition shown as "Blend 1" in Fig. 6 as a function of concentration;
Figure 14 shows a linear regression of inhibition of DPPH for the composition shown as "Blend 2" in Fig. 6 as a function of concentration;
Figure 15 shows a linear regression of inhibition of DPPH for the composition shown as "Blend 3" in Fig. 6 as a function of concentration;
Figure 16 shows a linear regression of inhibition of DPPH for the composition shown as "Blend 4" in Fig. 6 as a function of concentration;
Figure 17 shows a linear regression of inhibition of DPPH for the composition shown as "Blend 5" in Fig. 6 as a function of concentration;
Figure 18 shows UV-Vis spectrophotometric absorbance as a function of wavelength for the reaction of Example 4 carried out at 35°C at t=55min;
Figure 19 shows UV-Vis spectrophotometric absorbance as a function of wavelength at times from 0 to 55 minutes to illustrate the kinetics of the reaction of Example 4 carried out at 50°C;
Figure 20 shows UV-Vis spectrophotometric absorbance as a function of wavelength at times from 0 to 55 minutes to illustrate the kinetics of the reaction of Example 4 carried out at 60°C;
Figure 21 shows UV-Vis spectrophotometric absorbance as a function of wavelength at times from 0 to 55 minutes to illustrate the kinetics of the reaction of Example 4 carried out at 70°C;
Figure 22 shows UV-Vis spectrophotometric absorbance as a function of wavelength at times from 4 to 6.5 minutes to illustrate the kinetics of the reaction of Example 4 carried out at 85°C;
Figure 23 shows UV-Vis spectrophotometric absorbance as a function of wavelength at t=55min to compare the reaction kinetics from 50, 60 and 70°C for the reaction of Example 4;
Figure 24 shows UV-Vis spectrophotometric peak absorbance as a function of time at t=55min to compare the reaction kinetics from 50, 60 and 70°C for the reaction of Example 4;
Figure 25 shows UV-Vis spectrophotometric absorbance as a function of wavelength to illustrate the kinetics of the reaction of Example 4 under different blend concentrations;
Figure 26 is a scanning electron microscope (SEM) image of the organic polymer and metal nanoparticle produced according to Example 4;

Figure 27 is a scanning electron microscope (SEM) image of the organic polymer and metal nanoparticle produced according to Example 4;

Figure 28 is a scanning electron microscope (SEM) image of the organic polymer and metal nanoparticle produced according to Example 4;

Figure 29 is a scanning electron microscope (SEM) image of the metal nanoparticle produced according to Example 4;

Figure 30 is a scanning electron microscope (SEM) image of the metal nanoparticle produced according to Example 4;

Figure 31 is a scanning electron microscope (SEM) image of the metal nanoparticle produced according to Example 4;

Figure 32 is a scanning electron microscope (SEM) image of the metal nanoparticle produced according to Example 4;

Figure 33 is a scanning electron microscope (SEM) image of the metal nanoparticle produced according to Example 4;

Figure 34 is a scanning electron microscope (SEM) image of the metal nanoparticle produced according to Example 4;

Figure 35 is a scanning electron microscope (SEM) image of the metal nanoparticle produced according to Example 4;

Figure 36 is a scanning electron microscope (SEM) image of the metal nanoparticle produced according to Example 4;

Figure 37 is a scanning electron microscope (SEM) image of the metal nanoparticle produced according to Example 4;

Figure 38 is a scanning electron microscope (SEM) image of the metal nanoparticle produced according to Example 4;

Figure 39 is a scanning electron microscope (SEM) image of the organic polymer and metal nanoparticle produced according to Example 4;

Figure 40 is a scanning electron microscope (SEM) image of the metal nanoparticle produced according to Example 4;

Figure 41 is a scanning electron microscope (SEM) image of the metal nanoparticle produced according to Example 4;

Figure 42 is a scanning electron microscope (SEM) image of the metal nanoparticle produced according to Example 4;

Figure 43 is a scanning electron microscope (SEM) image of the metal nanoparticle produced according to Example 4;

Figure 44 is an energy dispersive X-ray spectroscopy image of the metal nanoparticle produced according to Example 4;

Figure 45 is an energy dispersive X-ray spectroscopy image of the metal nanoparticle produced according to Example 4;

Figure 46 is an energy dispersive X-ray spectroscopy image of the metal nanoparticle produced according to Example 4;

Figure 47 is an energy dispersive X-ray spectroscopy image of the metal nanoparticle produced according to Example 4;

Figure 48 is an energy dispersive X-ray spectroscopy image of the metal nanoparticle produced according to Example 4;

Figure 49 is an energy dispersive X-ray spectroscopy image of the metal nanoparticle produced according to Example 4;

Figure 50 is an energy dispersive X-ray spectroscopy image of the metal nanoparticle produced according to Example 4;

Figure 51 is an energy dispersive X-ray spectroscopy image of the metal nanoparticle produced according to Example 4;

Figure 52 is an energy dispersive X-ray spectroscopy image of the metal nanoparticle produced according to Example 4;

Figure 53 is an energy dispersive X-ray spectroscopy image of the metal nanoparticle produced according to Example 4;

Figure 54 is an energy dispersive X-ray spectroscopy image of the metal nanoparticle produced according to Example 4;

Figure 55 is an energy dispersive X-ray spectroscopy image of the metal nanoparticle produced according to Example 4;

Figure 56 is an energy dispersive X-ray spectroscopy image of the metal nanoparticle produced according to Example 4, showing evidence for a biopolymer protective layer (Carbon + Oxygen) on the silver nanoparticles;

Figure 57 is an energy dispersive X-ray spectroscopy image of the metal nanoparticle produced according to Example 4, showing evidence for a biopolymer protective layer (Carbon) on the silver nanoparticles;

Figure 58 is an energy dispersive X-ray spectroscopy image of the metal nanoparticle produced according to Example 4, showing evidence for a biopolymer protective layer (Oxygen) on the silver nanoparticles;

Figure 59 is a scanning electron microscope (SEM) image showing an outline of silver nanoparticles on a sampled area produced according to Example 4;

Figure 60 is a scanning electron microscope (SEM) image showing silver nanoparticles on a sampled area produced according to Example 4;

Figure 61 is a plot of frequency of nanoparticles versus diameter to show the silver nanoparticles particle size distribution on sampled area of Fig. 59;

Figure 62 is a plot of frequency of nanoparticles versus diameter to show the overall silver nanoparticles particle size distribution on multiple sampled areas;

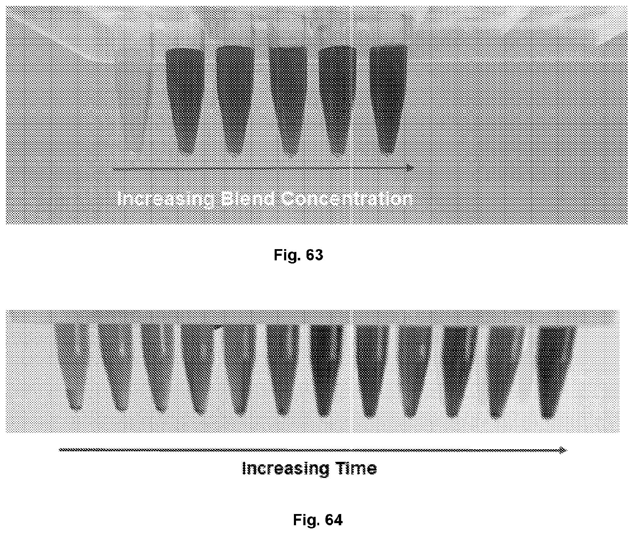

Figure 63 illustrates the colour of samples with increasing blend concentration;

Figure 64 illustrates the colour of samples with sampling at different times for 70°C Reaction for 55 minutes (every 5 minutes);

Figure 65 shows the comparison of Curly Kale (Set 1) and Watercress (Set 6) based blends according to comparative example 1;

Figure 66 shows the composition of Watercress Based Blends Composition according to comparative example 1;

Figure 67 is a UV/VIS spectrum of a silver nanoparticle, produced in accordance with Example 5, batch S1;

Figure 68 is a UV/VIS spectrum of a silver nanoparticle produced in accordance with Example 5, batch S2;

Figure 69 is a UV/VIS spectrum of a silver nanoparticle produced in accordance with Example 5, batch S3;

Figure 70 is a UV/VIS spectrum of a silver nanoparticle produced in accordance with Example 5, batch S4;

Figure 71 is a UV/VIS spectrum of a gold nanoparticle produced in accordance with Examples 6 and 7, batch S1;

Figure 72 is a UV/VIS spectrum of a gold nanoparticle produced in accordance with Examples 6 and 7, batch S2;

Figure 73 is a UV/VIS spectrum of a gold nanoparticle produced in accordance with Examples 6 and 7, batch S3;

Figure 74 is a plot of frequency of nanoparticles versus diameter to show the overall particle size distribution of a gold nanoparticle produced in accordance with Examples 6 and 7;

Figure 75 is a scanning electron microscope (SEM) image of the gold nanoparticle produced according to Examples 6 and 7;

Figure 76 is a scanning electron microscope (SEM) image of the gold nanoparticle produced according to Examples 6 and 7;

Figure 77 is a scanning electron microscope (SEM) image of the gold nanoparticle produced according to Examples 6 and 7;

Figure 78 is a scanning electron microscope (SEM) image of the gold nanoparticle produced according to Examples 6 and 7;

Figure 79 is a scanning electron microscope (SEM) image of the gold nanoparticle produced according to Examples 6 and 7;

Figure 80 is a scanning electron microscope (SEM) image of the gold nanoparticle produced according to Examples 6 and 7;

Figure 81 is a scanning electron microscope (SEM) image of the gold nanoparticle produced according to Examples 6 and 7;

Figure 82 is a scanning electron microscope (SEM) image of the gold nanoparticle produced according to Examples 6 and 7;

Figure 83 is an energy dispersive X-ray spectroscopy image of the gold nanoparticle produced according to Examples 6 and 7;

Figure 84 is an energy dispersive X-ray spectroscopy image of the gold nanoparticle produced according to Examples 6 and 7;

Figure 85 is an energy dispersive X-ray spectroscopy image of the metal nanoparticle produced according to Examples 6 and 7, mapping the gold atoms on the nanoparticles;

Figure 86 is an energy dispersive X-ray spectroscopy image of the metal nanoparticle produced according to Examples 6 and 7, mapping the carbon atoms on the biopolymer layer capping the nanoparticles;

Figure 87 is an energy dispersive X-ray spectroscopy image of the metal nanoparticle produced according to Examples 6 and 7, mapping the oxygen atoms on the biopolymer layer capping the nanoparticles;

Figure 88 is a scanning electron microscope (SEM) image of the gold nanoparticle produced according to Examples 6 and 7; and

Figure 89 is an energy dispersive X-ray Spectroscopy spectrum of the gold nanoparticles produced according to Examples 6 and 7.

## Detailed Description

## Definitions

In this specification the term "extract" means a substance obtained by extraction from a raw material. Typically the raw material is a naturally-occurring substance. In one embodiment the raw material is a plant, examples of which are described herein.

In this specification the term "biological active ingredient" means a substance obtained from a naturally-occurring source (typically an extract as defined above, and preferably a plant-based extract) which is capable of acting upon a metal ion to produce a metal nanoparticle as describe herein.

In one embodiment the biological active ingredient is a biological reducing agent (i.e. a substance obtained from a naturally-occurring source, typically a plant-based source) capable of reducing a metal ion to produce a metal nanoparticle, as described herein.

In one embodiment the biological active ingredient is a biological coating or capping agent. In one embodiment the biological coating or capping agent is a carbohydrate.

In this specification the term "antioxidant" means a substance which inhibits oxidation. In one embodiment an antioxidant is a substance which is capable of terminating a free-radical based chain reaction. In one embodiment the antioxidant is a naturally-occurring antioxidant, examples of which are described herein. In some aspects the term "antioxidant" is synonymous with "biological reducing agent" as defined above.

In this specification "nanoparticle" means a particle of matter between 1nm and 1$\mu$m in diameter. In one embodiment the nanoparticle is between 1nm and 500nm in diameter. In one embodiment the nanoparticle is between 1nm and 200nm in diameter. In one embodiment the nanoparticle is between 1nm and 100nm in diameter. In one embodiment the nanoparticle is between 1nm and 50nm in diameter. In one embodiment the nanoparticle is between 1nm and 20nm in diameter. In one embodiment the diameter of the nanoparticle is measured using ultraviolet-visible (UV-Vis) spectroscopy. In one embodiment the diameter of the nanoparticle is measured using scanning electron microscopy (SEM). In one embodiment the diameter of the nanoparticle is measured using transmission electron microscopy (TEM).

**Advantages and Surprising Findings**

It has surprisingly been found by the present inventors that that the compositions described herein, when used as reducing agents in the preparation of metal nanoparticles from a corresponding metal ion, exhibit activity which is greater than would have been expected from the additive reduction activity (for example, when tested using a DPPH assay), therefore suggesting a synergetic relationship between the different plants being used in the composition.

This surprising finding enables metal nanoparticles to be produced in a method which has a much lower environmental impact than known processes (including the current processes described in the prior art cited above). As the composition described herein has a much higher antioxidant activity (and therefore effectiveness as a biological reducing agent) and lower cost than extracts from single plants, and having a synergistic relationship as described above, much less material and energy usage is required in order to reduce the metal ions to metal nanoparticles. This is of particular importance when the process is scaled up.

The method of producing the metal nanoparticle according to the present invention, as it uses biological reducing agents, enables the use of less toxic and more environmentally friendly reagents compared with current manufacturing routes. It also minimises waste production throughout the process by including a recycle loop around the crystallisation in order to maximise process conversion at the point where the metal ions are introduced, thereby avoiding the discharge of large amounts of metal ions into the environment. Similarly, the addition of a recycle loop around the antioxidant extraction unit reduces waste and minimises environmental impact.

Unexpectedly, it has also been found that forming nanoparticles according to the method of the invention results in the nanoparticles this formed having a protective bio-layer (as described herein) thereon. This protects the nanoparticles without requiring additional capping agents to be added.

**Composition** - The following embodiments are not according to the invention and are present for illustration purposes only

The composition described herein is made from extracts of plants. Typically, the plants are naturally-occurring varieties. However, in the alternative the plant may be a genetically modified plant. In another alternative, the plant may be gene edited, for example using a technique such as CRISPR-Cas9.

In one aspect, there is provided a composition comprising extracts from:

(a) kale (*Brassica oleracea* Acephala group); and
(b) at least one other plant capable of producing a biological reducing agent.

A large number of plants are capable of producing antioxidants. Examples of such plants include, but are not limited to kale (*Brassica oleracea* Acephala group); artichoke (*Cynara cardunculus*); red cabbage (*Brassica oleracea var.* Capitata group *var. rubra*); oregano (*Origanum vulgare*); rosemary (*Salvia rosmarinus*); sage (*Salvia officinalis*); watercress (*Nasturtium officinale*); dog rose (*Rosa canina*); mint (*Mentha* spp.), especially spearmint (*Mentha spicata*) and peppermint (*Mentha x piperita*); thyme (*Thymus* spp. especially *Thymus cirtodorus, Thymus hera-barona, Thymus praecox, Thymus pseudolanugionsus, Tymus seryllum* and *Thymus vulgaris*); basil (*Osimum basilicum*); and spinach (*Spinacia oleracea*).

Therefore, in one embodiment, there is provided a composition comprising extracts from: (a) kale (*Brassica oleracea* Acephala group); and
(b) at least one other plant selected from the group consisting of: (i) artichoke *(Cynara cardunculus);* (ii) red cabbage *(Brassica oleracea var.* Capitata group *var. rubra);* (iii) oregano *(Origanum vulgare);* (iv) rosemary *(Salvia rosmarinus);* (v) sage *(Salvia officinalis);* (vi) watercress *(Nasturtium officinale);* (vii) dog rose (Rosa *canina);* (viii) mint *(Mentha* spp.); (ix) thyme *(Thymus* spp.); (x) basil *(Osimum basilicum);* and (xi) spinach *(Spinacia oleracea).*

In one embodiment, there is provided a composition comprising extracts from:

(a) kale *(Brassica oleracea* Acephala group); and
(b) at least one other plant selected from the group consisting of: (i) artichoke *(Cynara cardunculus);* (ii) cabbage *(Brassica oleracea* Capitata group *var. rubra);* (iii) oregano *(Origanum vulgare);* (iv) rosemary *(Salvia rosmarinus);* and (v) watercress *(Nasturtium officinale).*

In one embodiment, there is provided comprising extracts from:

(a) kale (*Brassica oleracea var.* Acephala group); and
(b) at least two other plants selected from the group consisting of:

(i) artichoke (*Cynara cardunculus*);
(ii) red cabbage (*Brassica oleracea var.* Capitata group *var. rubra*);
(iii) oregano (*Origanum vulgare*);
(iv) rosemary (*Salvia rosmarinus*);
(v) watercress (*Nasturtium officinale*); and.
(vi) sage (*Salvia officinalis*).

In one embodiment, there is provided comprising extracts from:

(a) kale (*Brassica oleracea var.* Acephala group); and
(b) at least two other plants selected from the group consisting of:

(i) artichoke (*Cynara cardunculus*);
(ii) red cabbage (*Brassica oleracea var.* Capitata group *var. rubra*);
(iii) oregano (*Origanum vulgare*);
(iv) rosemary (*Salvia rosmarinus*); and
(v) watercress (*Nasturtium officinale*).

In one embodiment, there is provided comprising extracts from:

(a) kale (*Brassica oleracea var.* Acephala group); and
(b) at least two other plants selected from the group consisting of:

(i) oregano (*Origanum vulgare*);
(ii) rosemary (*Salvia rosmarinus*);
(iii) watercress (*Nasturtium officinale*); and
(iv) sage (*Salvia officinalis*).

In one embodiment, there is provided a composition comprising extracts from:

(a) kale (*Brassica oleracea var.* Acephala group);
(b) artichoke (*Cynara cardunculus*); and
(c) red cabbage (*Brassica oleracea var.* Capitata group *var. rubra*).

In one embodiment, there is provided a composition comprising extracts from:

(a) kale (*Brassica oleracea var.* Acephala group);
(b) artichoke (*Cynara cardunculus*); and
(c) oregano (*Origanum vulgare*).

In one embodiment, there is provided a composition comprising extracts from:

(a) kale (*Brassica oleracea var.* Acephala group);
(b) red cabbage (*Brassica oleracea var.* Capitata group *var. rubra*); and

(c) oregano (*Origanum vulgare*).

In one embodiment, there is provided a composition comprising extracts from:

(a) kale (*Brassica oleracea var.* Acephala group); and
(b) at least two other plants selected from the group consisting of:

(i) artichoke (*Cynara cardunculus*);
(ii) red cabbage (*Brassica oleracea var.* Capitata group *var. rubra*);
(iii) oregano (*Origanum vulgare*);
(iv) rosemary (*Salvia rosmarinus*); and
(v) watercress (*Nasturtium officinale*).

In one embodiment, there is provided a composition comprising extracts from:

(a) kale (B*rassica oleracea var.* Acephala group); and
(b) at least three other plants selected from the group consisting of:

(i) artichoke (*Cynara cardunculus*);
(ii) red cabbage (*Brassica oleracea var.* Capitata group *var. rubra*);
(iii) oregano (*Origanum vulgare*);
(iv) rosemary (*Salvia rosmarinus*); and
(v) watercress (*Nasturtium officinale*).

In one embodiment, there is provided a composition comprising extracts from:

(a) kale (*Brassica oleracea var.* Acephala group);
(b) red cabbage (*Brassica oleracea var.* Capitata group *var. rubra*);
(c) oregano (*Origanum vulgare*); and
(d) rosemary (*Salvia rosmarinus*).

In one embodiment, there is provided a composition comprising extracts from:

(a) kale (*Brassica oleracea var.* Acephala group); and
(b) at least three other plants selected from the group consisting of:

(i) artichoke (*Cynara cardunculus*);
(ii) red cabbage (*Brassica oleracea var.* Capitata group *var. rubra*); and
(iii) rosemary (*Salvia rosmarinus*).

In one embodiment, there is provided a composition comprising extracts from:

(a) kale (*Brassica oleracea var.* Acephala group); and
(b) at least four other plants selected from the group consisting of:

(i) artichoke (*Cynara cardunculus*);
(ii) red cabbage (*Brassica oleracea var.* Capitata group *var. rubra*);
(iii) oregano (*Origanum vulgare*);
(iv) rosemary (*Salvia rosmarinus*); and
(v) watercress (*Nasturtium officinale*).

In one embodiment, there is provided a composition comprising extracts from:

(a) kale (*Brassica oleracea var.* Acephala group);
(b) artichoke (*Cynara cardunculus*);
(c) oregano (*Origanum vulgare*);
(d) rosemary (*Salvia rosmarinus*); and
(e) watercress (*Nasturtium officinale*).

In one embodiment, there is provided a composition comprising extracts from:

(a) kale (*Brassica oleracea var.* Acephala group); and
(b) red cabbage (*Brassica oleracea var.* Capitata group *var. rubra*);
(c) oregano (*Origanum vulgare*);
(d) rosemary (*Salvia rosmarinus*); and
(e) watercress (*Nasturtium officinale*).

The composition disclosed herein typically contains extract of kale (*Brassica oleracea var.* Acephala group). In one embodiment the composition contains at least 5% extract of kale by mass of the total mass of the composition. In one embodiment the composition contains at least 10% extract of kale by mass of the total mass of the composition. In one embodiment the composition contains at least 15% extract of kale by mass of the total mass of the composition. In one embodiment the composition contains at least 20% extract of kale by mass of the total mass of the composition. In one embodiment the composition contains at least 25% extract of kale by mass of the total mass of the composition. In one embodiment the composition contains at least 30% extract of kale by mass of the total mass of the composition. In one embodiment the composition contains at least 35% extract of kale by mass of the total mass of the composition. In one embodiment the composition contains at least 40% extract of kale by mass of the total mass of the composition. In one embodiment the composition contains at least 45% extract of kale by mass of the total mass of the composition. In one embodiment the composition contains at least 50% extract of kale by mass of the total mass of the composition. In one embodiment the composition contains at least 55% extract of kale by mass of the total mass of the composition. In one embodiment the composition contains at least 60% extract of kale by mass of the total mass of the composition. In one embodiment the composition contains at least 65% extract of kale by mass of the total mass of the composition. In one embodiment the composition contains at least 70% extract of kale by mass of the total mass of the composition. In one embodiment the composition contains at least 75% extract of kale by mass of the total mass of the composition. In one embodiment the composition contains at least 80% extract of kale by mass of the total mass of the composition.

In one embodiment the composition contains at most 95% extract of kale by mass of the total mass of the composition. In one embodiment the composition contains at most 90% extract of kale by mass of the total mass of the composition. In one embodiment the composition contains at most 85% extract of kale by mass of the total mass of the composition. In one embodiment the composition contains at most 80% extract of kale by mass of the total mass of the composition. In one embodiment the composition contains at most 75% extract of kale by mass of the total mass of the composition. In one embodiment the composition contains at most 70% extract of kale by mass of the total mass of the composition. In one embodiment the composition contains at most 65% extract of kale by mass of the total mass of the composition. In one embodiment the composition contains at most 60% extract of kale by mass of the total mass of the composition. In one embodiment the composition contains at most 55% extract of kale by mass of the total mass of the composition. In one embodiment the composition contains at most 50% extract of kale by mass of the total mass of the composition. In one embodiment the composition contains at most 45% extract of kale by mass of the total mass of the composition. In one embodiment the composition contains at most 40% extract of kale by mass of the total mass of the composition. In one embodiment the composition contains at most 35% extract of kale by mass of the total mass of the composition. In one embodiment the composition contains at most 30% extract of kale by mass of the total mass of the composition. In one embodiment the composition contains at most 25% extract of kale by mass of the total mass of the composition. In one embodiment the composition contains at most 20% extract of kale by mass of the total mass of the composition. In one embodiment the composition contains at most 15% extract of kale by mass of the total mass of the composition. In one embodiment the composition contains at most 10% extract of kale by mass of the total mass of the composition.

In one embodiment the composition contains extract of kale in an amount of 5% to 95% by mass of the total mass of the composition. In one embodiment the composition contains extract of kale in an amount of 10% to 90% by mass of the total mass of the composition. In one embodiment the composition contains extract of kale in an amount of 20% to 95% by mass of the total mass of the composition.

In one embodiment the composition contains extract of kale in an amount of 30% to 90% by mass of the total mass of the composition. In one embodiment the composition contains extract of kale in an amount of 35% to 85% by mass of the total mass of the composition. In one embodiment the composition contains extract of kale in an amount of 40% to 80% by mass of the total mass of the composition.

In one embodiment the composition contains extract of kale in an amount of 45% to 75% by mass of the total mass of the composition. In one embodiment the composition contains extract of kale in an amount of 50% to 70% by mass of the total mass of the composition.

In one embodiment, the composition disclosed herein comprises extract of artichoke (*Cynara cardunculus*). In one embodiment the composition contains at least 1% extract of artichoke by mass of the total mass of the composition. In one embodiment the composition contains at least 2.5% extract of artichoke by mass of the total mass of the composition.

In one embodiment the composition contains at least 5% extract of artichoke by mass of the total mass of the composition. In one embodiment the composition contains at least 7.5% extract of artichoke by mass of the total mass of the composition. In one embodiment the composition contains at least 10% extract of artichoke by mass of the total mass of the composition. In one embodiment the composition contains at least 12.5% extract of artichoke by mass of the total mass of the composition. In one embodiment the composition contains at least 15% extract of artichoke by mass of the total mass of the composition.

In one embodiment the composition contains at most 40% extract of artichoke by mass of the total mass of the composition. In one embodiment the composition contains at most 35% extract of artichoke by mass of the total mass of the composition. In one embodiment the composition contains at most 30% extract of artichoke by mass of the total mass of the composition. In one embodiment the composition contains at most 25% extract of artichoke by mass of the total mass of the composition. In one embodiment the composition contains at most 20% extract of artichoke by mass of the total mass of the composition. In one embodiment the composition contains at most 15% extract of artichoke by mass of the total mass of the composition. In one embodiment the composition contains at most 12.5% extract of artichoke by mass of the total mass of the composition. In one embodiment the composition contains at most 10% extract of artichoke by mass of the total mass of the composition. In one embodiment the composition contains at most 7.5% extract of artichoke by mass of the total mass of the composition. In one embodiment the composition contains at most 5% extract of artichoke by mass of the total mass of the composition. In one embodiment the composition contains at most 2.5% extract of artichoke by mass of the total mass of the composition. In one embodiment the composition contains at most 1% extract of artichoke by mass of the total mass of the composition.

In one embodiment the composition contains extract of artichoke in an amount of 0.5% to 40% by mass of the total mass of the composition. In one embodiment the composition contains extract of artichoke in an amount of 10% to 90% by mass of the total mass of the composition. In one embodiment the composition contains extract of artichoke in an amount of 1% to 35% by mass of the total mass of the composition. In one embodiment the composition contains extract of artichoke in an amount of 2.5% to 30% by mass of the total mass of the composition. In one embodiment the composition contains extract of artichoke in an amount of 5% to 25% by mass of the total mass of the composition. In one embodiment the composition contains extract of artichoke in an amount of 10% to 20% by mass of the total mass of the composition.

In one embodiment, the composition disclosed herein comprises extract of red cabbage (*Brassica oleracea var.* Capitata group *var. rubra*). In one embodiment the composition contains at least 1% extract of red cabbage by mass of the total mass of the composition. In one embodiment the composition contains at least 2.5% extract of red cabbage by mass of the total mass of the composition. In one embodiment the composition contains at least 5% extract of red cabbage by mass of the total mass of the composition. In one embodiment the composition contains at least 10% extract of red cabbage by mass of the total mass of the composition. In one embodiment the composition contains at least 15% extract of red cabbage by mass of the total mass of the composition. In one embodiment the composition contains at least 20% extract of red cabbage by mass of the total mass of the composition. In one embodiment the composition contains at least 25% extract of red cabbage by mass of the total mass of the composition.

In one embodiment the composition contains at most 50% extract of red cabbage by mass of the total mass of the composition. In one embodiment the composition contains at most 45% extract of red cabbage by mass of the total mass of the composition. In one embodiment the composition contains at most 40% extract of red cabbage by mass of the total mass of the composition. In one embodiment the composition contains at most 35% extract of red cabbage by mass of the total mass of the composition. In one embodiment the composition contains at most 30% extract of red cabbage by mass of the total mass of the composition. In one embodiment the composition contains at most 25% extract of red cabbage by mass of the total mass of the composition. In one embodiment the composition contains at most 20% extract of red cabbage by mass of the total mass of the composition. In one embodiment the composition contains at most 15% extract of red cabbage by mass of the total mass of the composition. In one embodiment the composition contains at most 10% extract of red cabbage by mass of the total mass of the composition. In one embodiment the composition contains at most 5% extract of red cabbage by mass of the total mass of the composition. In one embodiment the composition contains at most 2.5% extract of red cabbage by mass of the total mass of the composition. In one embodiment the composition contains at most 1% extract of red cabbage by mass of the total mass of the composition.

In one embodiment the composition contains extract of red cabbage in an amount of 0.5% to 50% by mass of the total mass of the composition. In one embodiment the composition contains extract of red cabbage in an amount of 1% to 40% by mass of the total mass of the composition. In one embodiment the composition contains extract of red cabbage in an amount of 1% to 35% by mass of the total mass of the composition. In one embodiment the composition contains extract of red cabbage in an amount of 2.5% to 30% by mass of the total mass of the composition. In one embodiment the composition contains extract of red cabbage in an amount of 5% to 25% by mass of the total mass of the composition. In one embodiment the composition contains extract of red cabbage in an amount of 10% to 20% by mass of the total mass of the composition.

In one embodiment, the composition disclosed herein comprises extract of oregano *(Origanum vulgare).* In one embodiment the composition contains at least 0.5% extract of oregano by mass of the total mass of the composition. In one embodiment the composition contains at least 1% extract of oregano by mass of the total mass of the composition. In one embodiment the composition contains at least 2% extract of oregano by mass of the total mass of the composition. In one embodiment the composition contains at least 3% extract of oregano by mass of the total mass of the composition. In one embodiment the composition contains at least 5% extract of oregano by mass of the total mass of the composition. In one embodiment the composition contains at least 7.5% extract of oregano by mass of the total mass of the composition. In one embodiment the composition contains at least 10% extract of oregano by mass of the total mass of the composition.

In one embodiment the composition contains at most 30% extract of oregano by mass of the total mass of the composition. In one embodiment the composition contains at most 25% extract of oregano by mass of the total mass of the composition. In one embodiment the composition contains at most 20% extract of oregano by mass of the total mass of the composition. In one embodiment the composition contains at most 15% extract of oregano by mass of the total mass of the composition. In one embodiment the composition contains at most 10% extract of oregano by mass of the total mass of the composition. In one embodiment the composition contains at most 5% extract of oregano by mass of the total mass of the composition. In one embodiment the composition contains at most 2.5% extract of oregano by mass of the total mass of the composition. In one embodiment the composition contains at most 1% extract of oregano by mass of the total mass of the composition.

In one embodiment the composition contains extract of oregano in an amount of 0.5% to 30% by mass of the total mass of the composition. In one embodiment the composition contains extract of oregano in an amount of 1% to 20% by mass of the total mass of the composition. In one embodiment the composition contains extract of oregano in an amount of 2% to 15% by mass of the total mass of the composition. In one embodiment the composition contains extract of oregano in an amount of 5% to 10% by mass of the total mass of the composition.

In one embodiment, the composition disclosed herein comprises extract of rosemary (*Salvia rosmarinus*). In one embodiment the composition contains at least 1% extract of rosemary by mass of the total mass of the composition. In one embodiment the composition contains at least 2.5% extract of rosemary by mass of the total mass of the composition. In one embodiment the composition contains at least 5% extract of rosemary by mass of the total mass of the composition. In one embodiment the composition contains at least 10% extract of rosemary by mass of the total mass of the composition. In one embodiment the composition contains at least 15% extract of rosemary by mass of the total mass of the composition. In one embodiment the composition contains at least 20% extract of rosemary by mass of the total mass of the composition. In one embodiment the composition contains at least 25% extract of rosemary by mass of the total mass of the composition.

In one embodiment the composition contains at most 50% extract of rosemary by mass of the total mass of the composition. In one embodiment the composition contains at most 45% extract of rosemary by mass of the total mass of the composition. In one embodiment the composition contains at most 40% extract of rosemary by mass of the total mass of the composition. In one embodiment the composition contains at most 35% extract of rosemary by mass of the total mass of the composition. In one embodiment the composition contains at most 30% extract of rosemary by mass of the total mass of the composition. In one embodiment the composition contains at most 25% extract of rosemary by mass of the total mass of the composition. In one embodiment the composition contains at most 20% extract of rosemary by mass of the total mass of the composition. In one embodiment the composition contains at most 15% extract of rosemary by mass of the total mass of the composition. In one embodiment the composition contains at most 10% extract of rosemary by mass of the total mass of the composition. In one embodiment the composition contains at most 5% extract of rosemary by mass of the total mass of the composition. In one embodiment the composition contains at most 2.5% extract of rosemary by mass of the total mass of the composition. In one embodiment the composition contains at most 1% extract of rosemary by mass of the total mass of the composition.

In one embodiment the composition contains extract of rosemary in an amount of 0.5% to 50% by mass of the total mass of the composition. In one embodiment the composition contains extract of rosemary in an amount of 1% to 40% by mass of the total mass of the composition. In one embodiment the composition contains extract of rosemary in an amount of 1% to 35% by mass of the total mass of the composition. In one embodiment the composition contains extract of rosemary in an amount of 2.5% to 30% by mass of the total mass of the composition. In one embodiment the composition contains extract of rosemary in an amount of 5% to 25% by mass of the total mass of the composition. In one embodiment the composition contains extract of rosemary in an amount of 10% to 20% by mass of the total mass of the composition.

In one embodiment, the composition disclosed herein comprises extract of watercress (*Nasturtium officinale*). In one embodiment the composition contains at least 0.5% extract of watercress by mass of the total mass of the composition. In one embodiment the composition contains at least 1% extract of watercress by mass of the total mass of the composition. In one embodiment the composition contains at least 2% extract of watercress by mass of the total mass of the composition. In one embodiment the composition contains at least 3% extract of watercress by mass of the total mass of the composition. In one embodiment the composition contains at least 5% extract of watercress by mass of the total mass of the composition. In one embodiment the composition contains at least 7.5% extract of watercress by mass of the total mass of the composition. In one embodiment the composition contains at least 10% extract of watercress by mass of the total mass of the composition.

In one embodiment the composition contains at most 30% extract of watercress by mass of the total mass of the composition. In one embodiment the composition contains at most 25% extract of watercress by mass of the total mass of the composition. In one embodiment the composition contains at most 20% extract of watercress by mass of the total mass of the composition. In one embodiment the composition contains at most 15% extract of watercress by mass of the total mass of the composition. In one embodiment the composition contains at most 10% extract of watercress by mass of the total mass of the composition. In one embodiment the composition contains at most 5% extract of watercress by mass of the total mass of the composition. In one embodiment the composition contains at most 2.5% extract of watercress by mass of the total mass of the composition. In one embodiment the composition contains at most 1% extract of watercress by mass of the total mass of the composition.

In one embodiment the composition contains extract of watercress in an amount of 0.5% to 30% by mass of the total mass of the composition. In one embodiment the composition contains extract of watercress in an amount of 1% to 20% by mass of the total mass of the composition. In one embodiment the composition contains extract of watercress in an amount of 2% to 15% by mass of the total mass of the composition. In one embodiment the composition contains extract of watercress in an amount of 5% to 10% by mass of the total mass of the composition.

In one embodiment the composition contains extracts from the following:

(i) kale (*Brassica oleracea var.* Acephala group), in an amount of 60% to 90% by mass, as a proportion of the total mass of the composition;
(ii) artichoke (*Cynara cardunculus*), in an amount of 10% to 20% by mass, as a proportion of the total mass of the composition; and
(iii) oregano (*Origanum vulgare*) in an amount of 5% to 15% by mass, as a proportion of the total mass of the composition.

In one embodiment the composition contains extracts from the following:

(i) kale (*Brassica oleracea var.* Acephala group), in an amount of 60% to 90% by mass, as a proportion of the total mass of the composition;
(ii) red cabbage (*Brassica oleracea var.* Capitata group *var. rubra*), in an amount of 5% to 15% by mass, as a proportion of the total mass of the composition; and
(iii) oregano (*Origanum vulgare*) in an amount of 5% to 15% by mass, as a proportion of the total mass of the composition;.

In one embodiment the composition contains extracts from the following:

(i) kale (*Brassica oleracea var.* Acephala group), in an amount of 50% to 80% by mass, as a proportion of the total mass of the composition;
(ii) artichoke (*Cynara cardunculus*), in an amount of 15% to 25% by mass, as a proportion of the total mass of the composition;
(iii) oregano (*Origanum vulgare*) in an amount of 5% to 20% by mass, as a proportion of the total mass of the composition;
(iv) rosemary (*Salvia rosmarinus*), in an amount of 0.5% to 5% by mass, as a proportion of the total mass of the composition; and
(v) watercress (*Nasturtium officinale*), in an amount of 1% to 10% by mass, as a proportion of the total mass of the composition.

In one embodiment the composition contains extracts from the following:

(i) kale (*Brassica oleracea var.* Acephala group), in an amount of 50% to 70% by mass, as a proportion of the total mass of the composition;
(ii) red cabbage (*Brassica oleracea var.* Capitata group *var. rubra*), in an amount of 5% to 15% by mass, as a proportion of the total mass of the composition;
(iii) oregano (*Origanum vulgare*) in an amount of 1% to 15% by mass, as a proportion of the total mass of the composition;
(iv) rosemary (*Salvia rosmarinus*), in an amount of 15% to 35% by mass, as a proportion of the total mass of the composition; and

In one embodiment the composition contains extracts from the following:

(i) kale (*Brassica oleracea var.* Acephala group), in an amount of 10% to 50% by mass, as a proportion of the total mass of the composition;

(ii) red cabbage (*Brassica oleracea var.* Capitata group *var. rubra*), in an amount of 5% to 30% by mass, as a proportion of the total mass of the composition;

(iii) oregano (*Origanum vulgare*) in an amount of 0.5% to 10% by mass, as a proportion of the total mass of the composition;

(iv) rosemary (*Salvia rosmarinus*), in an amount of 2.5% to 25% by mass, as a proportion of the total mass of the composition; and

(v) watercress (*Nasturtium officinale*), in an amount of 10% to 30% by mass, as a proportion of the total mass of the composition.

The composition disclosed herein contains antioxidants which act as biological reducing agents. In one embodiment the antioxidant is selected from the group consisting of ascorbic acid, beta-carotene; retinol (Vitamin A); a phenolic compound (especially a polyphenol, such as a flavonoid or epicatechin); lutein; all-*trans*-β-carotene; 9-*cis*-β-carotene; neoxanthin; violaxanthin; narirutin; apigenin 7-rutinoside; cynarin; 1-caffeoylquinic acid; luteolin; 7-apiosyl-(1->2)-glucoside; cynaroside; caffeic acid; a tannin; chlorogenic acid; luteolin-7-rutinoside; an anthocyanin, such as cyanidin-3-diglucoside-5-glucosides; kaempferol; p-coumaric acid; ferrulic acid; myricetin; resveratrol; rosmarinic acid; carnosol; hesperidin; rosmanol; epirosmanol; rosmadial; carnosic acid; methyl carnosate; gallic acid;quercetin-3-O-rutinoside; dicaffeoyltartaric acid; isorhamnetin; cyanidin-3-glucoside; luteolin-7-O-glucoside; quercetin-3-O-rhamnoside; and gluconasturtiin.

When the composition contains kale, any or all of the following antioxidants may be present in the composition, in varying proportions: ascorbic acid (Vitamin C); beta-Carotene; retinol (Vitamin A); phenolic Compounds (polyphenols); lutein; all-trans-β-carotene; 9-*cis*-β-carotene; neoxanthin; violaxanthin.

When the composition contains artichoke, any or all of the following antioxidants may be present in the composition, in varying proportions: narirutin; apigenin 7-rutinoside; cynarin; 1-caffeoylquinic acid; luteolin 7-apiosyl-(1->2)-glucoside; cynaroside; flavonoids; caffeic acid; tannins; chlorogenic acid; luteolin-7-rutinoside.

When the composition contains red cabbage, any or all of the following antioxidants may be present in the composition, in varying proportions: anthocyanins such as cyanidin-3-diglucoside-5-glucosides; kaempferol; vitamin C; vitamin A; lutein.

When the composition contains oregano, any or all of the following antioxidants may be present in the composition, in varying proportions: chlorogenic acid; epicatechin; p-coumaric acid; caffeic acid; ferrulic acid; rosemary acid; myricetin; resveratrol.

When the composition contains rosemary, any or all of the following antioxidants may be present in the composition, in varying proportions: rosmarinic acid; carnosic acid; carnosol; hesperidin.

When the composition contains sage, any or all of the following antioxidants may be present in the composition, in varying proportions: rosmanol; epirosmanol; carnosol; rosmadial; carnosic acid; methyl carnosate.

When the composition contains watercress, any or all of the following antioxidants may be present in the composition, in varying proportions: gallic acid; chlorogenic acid; caffeic acid; quercetin-3-O-rutinoside; dicaffeoyltartaric acid; isorhamnetin; cyanidin-3-glucoside; luteolin-7-O-glucoside; quercetin-3-O-rhamnoside; gluconasturtiin.

In one embodiment the antioxidant is ascorbic acid.

In one embodiment the composition comprises at least 5 mmol antioxidants per 100g of the composition. In one embodiment the composition comprises at least 15 mmol antioxidants per 100g of the composition.

**Method of Preparing Composition** - The following embodiments are not according to the invention and are present for illustration purposes only

The compositions disclosed herein may be prepared by treating the plants such that the active biological reducing agents (antioxidant) components of the composition are extracted from the plant into the solution.

In one embodiment, the method comprising one or more of the following steps:

(a) providing a preparation of the plants as defined above; and

(b) extracting biological active ingredients from the combined preparation using a solvent.

In one embodiment, the plant preparation step comprises drying the plants. In one embodiment, the plants are blended before the drying step. In one embodiment, the individual plants are dried before the particle size reduction step.

The temperature at which the drying step is carried out is typically from room temperature to 80°C, preferably from 30 to 50°C, more preferably 40°C.

The pressure at which the drying step is carried out is typically from 0.2 to 10 bar, preferably from 0.5 to 2 bar, and more preferably 0.9 to 1.1 bar.

The time for which the drying step is carried out is typically from 10 minutes to 72 hours, preferably from 1 to 48 hours, and more preferably 12 to 36 hours.

In one embodiment, the plant preparation step comprises reducing the particle size of the dried plants. This step can be carried out using any method or apparatus suitable for carrying out this task. Typically it is carried out in a blender. Typically, the particle size to which the plants are reduced ranges from 10 $\mu$m to 1 mm, preferably 20 to 200 $\mu$m, more preferably 100 $\mu$m.

In one embodiment, the extraction step is carried out by leaching. The leaching process permits extraction of the biological active ingredients. Typically, the process comprises the step of dissolution of the biological active ingredients from the dried plant particles into the solvent. The active ingredients typically undergo mass transfer diffusion from the inner pores of the particles towards the solid-liquid interface, followed by mass transfer diffusion from the solid-liquid interface into the bulk solution.

The solvent used in the extraction step may comprise any solvent which is capable of dissolving the biological active ingredients. Examples of suitable solvents include water; ethers such as diethyl ether and 1,2-dimethoxyethane; hydrocarbons such as hexane; halogenated hydrocarbons such as dichloromethane and chloroform; and mixtures thereof. However, it is preferred that the solvent used in the extraction step is water, as it is capable of dissolving oxygenated active ingredients such as ascorbic acid which are present in many of the blended products while avoiding the toxicity and environmental effect of some organic solvents.

The temperature at which the extraction step is carried out depends on the nature of the plants, the solvent, and the biological agents requiring extraction. However, the temperature is typically from room temperature to the boiling point of the solvent, preferably from 30 to 70°C, more preferably from 40 to 60°C.

The pressure at which the extraction step is carried out depends on the nature of the plants, the solvent, and the biological agents requiring extraction. However, the pressure is typically from 0.2 to 10 bar, preferably from 0.5 to 2 bar, and more preferably 0.9 to 1.1 bar.

The time for which the extraction step is carried out is typically from 1 minutes to 72 hours, preferably from 2 minutes to 4 hours, and more preferably 5 to 60 minutes.

Typically the extraction process is carried out with stirring.

In one embodiment, after the extraction step, the solution may be decanted to separate the solution from the solid matter. The decanter may form three phases (Scum, Clarified Liquid, Sludge). The sludge may be recycled back to the mixer for further processing to extract the maximum amount of biological active ingredients, until the concentration in the solid particles becomes minimal. At this point the depleted plant particles may be disposed from the process.

In one embodiment, after decantation but before filtration, the biological active ingredients may be recovered and/or the cells broken by passage of the filtrate through a hydrocyclone. This also uses centrifugal forces to remove solids, but is carried out on a continuous basis.

In one embodiment, after the extraction step, or after the decanting step, the composition may be filtered to produce a filtrate and a residue. Depending on the solids content, the filtration may be carried out under pressure, under vacuum, or a combination thereof. The pressure at which the filtration step is carried out depends on the nature of the plants, the solvent, and the biological agents requiring extraction. The pressure at which the filtration step is carried out is typically from 0.1 to 100 bar, preferably from 0.3 to 10 bar, and more preferably 0.3 to 5 bar. In one embodiment the filtration step is carried out at a pressure from 0.001 to 0.1 bar.

In one embodiment, after the extraction step, or after the decanting step, the composition may be filtered through a screen to remove larger particles.

In one embodiment, after the filtration step, the filtrate may be centrifuged. Centrifugation allows further cells to be broken down, removes smaller particles allows a greater amount of the biological active ingredient to be dissolved. In one embodiment, the centrifugation is carried out at 500 to 20,000 rpm. In one embodiment, the centrifugation is carried out at 500 to 15,000 rpm. In one embodiment, the centrifugation is carried out at 300 to 5000 rpm. In one embodiment, the centrifugation is carried out at 100 to 3000 rpm.

In one embodiment, the centrifugation is carried out at 2500 rpm. This centrifugal speed is particularly preferred as it allows the removal of many broken cells in the precipitate which would interfere with the nucleation of nanoparticles and allows large amounts of impurities in the crystals. By eliminating the cells from the filtrate, it is possible to achieve nanoparticles which have particle size distributions and are more spherical.

In one embodiment, the centrifugation is carried out for 10 seconds to 1 hour. In one embodiment, the centrifugation is carried out for 30 seconds to 30 minutes.

In one embodiment, the centrifugation is carried out for 1 to 20 minutes. In one embodiment, the centrifugation is carried out for 10 minutes.

In an alternative embodiment, the biological active ingredients may be recovered from the solution by chromatography. This may be thin layer chromatography, column chromatography, liquid chromatography, in particular high pressure liquid chromatography, or gas chromatography.

In an alternative embodiment, the biological active ingredients may be recovered from the solution by passage of the solution through a membrane. Examples of such techniques include micro and nano filtration techniques.

In an alternative embodiment, the biological active ingredients may be recovered from the solution by precipitation or crystallization. In an alternative embodiment, the biological active ingredients may be recovered from the solution by liquid-liquid extraction. In an alternative embodiment, the biological active ingredients may be recovered from the solution by drying. In an alternative embodiment, the biological active ingredients may be recovered from the solution by osmotic shock.

In an alternative embodiment, the biological active ingredients may be recovered from the solution by electrophoresis. All of these techniques are well known to those skilled in the art.

**Metal Nanoparticle** - The following embodiments are not according to the invention and are present for illustration purposes only

Also disclosed herein are metal nanoparticles obtained or obtainable by the process of the invention.

Unexpectedly, it has been found that forming nanoparticles according to the method of the invention results in the nanoparticles this formed having a protective bio-layer thereon. This protects the nanoparticles without requiring additional capping agents to be added.

Therefore, in another aspect, there is provided a metal nanoparticle, provided with a coating layer. In one embodiment the coating layer contains carbon. In one embodiment the coating layer contains oxygen. In one embodiment the coating layer contains nitrogen. In one embodiment the coating layer contains sulphur. In one embodiment the coating layer comprises a bio-polymer. In one embodiment the coating layer comprises a carbohydrate. In one embodiment the coating layer contains compounds identified above as antioxidants in the biological reducing agents of the composition of the invention as described and exemplified above, or compounds produced therefrom.

The nanoparticles may comprise a single metal or a mixture of metals. In one embodiment the nanoparticles comprise a single metal. In one embodiment the nanoparticles comprise a mixture of metals.

The metal from which the nanoparticle is formed may be any metal capable of forming nanoparticles and which is stable to the process described above. Examples of suitable metals include, but are not limited to gold, silver, copper, nickel, platinum, iron, zinc, gadolinium, titanium, cerium, thallium, manganese, aluminium, zirconium, chromium, palladium and cobalt, or any combination thereof.

In one embodiment, the metal is selected from the group consisting of silver, gold, platinum, aluminium, zirconium, and iron, or any combination thereof.

In one embodiment, the metal is selected from the group consisting of silver, gold and copper, or any combination thereof. In one embodiment, the metal is silver. In one embodiment, the metal is gold.

In one embodiment, the nanoparticle is a multi-layer nanoparticle. Typically such multi-layer nanoparticles comprise an inner core and an outer shell. The metals comprising the core and shell may be the same or different, and may be any of the metals listed above. By way of example, the nanoparticle may comprise an iron core and a silver outer shell.

The metal nanoparticles disclosed herein may be formed into a variety of shapes, depending on the precise nature of the process used to form them and their intended use. Typical examples of suitable shapes include spheres, ovoids, cylinders, rods, cubes and cuboids, prisms (e.g. triangular, pentagonal or hexagonal), cones and pyramids.

**Method of Producing Metal Nanoparticle**

The invention provides a method of producing a metal nanoparticle having a diameter between 1nm and 1 μm, the method comprising:

(a) providing a dissolved metal ion; and
(b) contacting the dissolved metal ion with a composition comprising extracts from:

(a) kale (*Brassica oleracea* Acephala group) and oregano (*Origanum vulgare*); and
(b) at least one other plant selected from the group consisting of:

(i) artichoke (*Cynara cardunculus*);
(ii) red cabbage (*Brassica oleracea var.* Capitata group *var. rubra*);
(iii) rosemary (*Salvia rosmarinus*);
(iv) sage (*Salvia officinalis*);
(v) watercress (*Nasturtium officinale*);

such that the dissolved metal ion is reduced to form the metal nanoparticle.

Typically, the dissolved metal ion is present in the form of a solution of a metal salt. It is preferred that the salt contains a counter-ion which has low toxicity to humans and low environmental impact. Examples of suitable salts include chloride, sulphate, carbonate, gluconate, and acetate. In one embodiment, the metal salt is an organic metal salt. In one embodiment the metal salt is an acetate salt.

In one embodiment, the dissolved metal ion is present in the form of a solvate of a metal salt. It is preferred that the solvent which forms the solvate has low toxicity to humans and low environmental impact. In one embodiment, the dissolved metal ion is present in the form of a hydrate, where the solvent is water.

When the metal is silver, preferred salts include silver chloride, silver carbonate and silver acetate, or a solvate of any thereof. When the metal is copper, preferred salts include copper (II) sulphate and copper (II) gluconate, or a solvate of any thereof. When the metal is gold, preferred salts include gold (I) chloride and gold (III) chloride, or a solvate of any thereof.

The solvent used in the contacting step may comprise any solvent which is capable of dissolving the biological active ingredients. Examples of suitable solvents include water; and alcohols such as methanol, ethanol and isopropanol; and mixtures thereof. However, it is preferred that the solvent used in the contacting step is water, as it is capable of dissolving both the metal ion and oxygenated biological reducing agents such as ascorbic acid which are present in many of the blended products while avoiding the toxicity and environmental effect of some organic solvents.

The contacting step may be carried out by any means known to those skilled in the art to be capable of producing metal nanoparticles from dissolved metal ions. It is preferred that the composition is added to a solution of metal ions. In an alternative embodiment a solution of metal ions is added to the composition.

In one embodiment a smaller volume of the composition of higher concentration is added to a larger volume of solution of metal ions of lower concentration. In one embodiment the volume of the solution of metal ions used is 2 to 50 times the volume of the composition. In one embodiment the volume of the solution of metal ions used is 5 to 20 times the volume of the composition.

It is preferred that the solution of metal ions is pre-heated before addition of the composition. It has been found that nucleation to produce a nanoparticle is essentially instantaneous when this method step is used. In one embodiment the solution of metal ions is pre-heated to a temperature from **40** to 100°C. In one embodiment solution of metal ions is pre-heated to a temperature from 60 to 80°C.

Typically, the concentration of metal ions used in the contacting step is between 0.1 to 5 g/L, preferably 0.5 to 1.5 g/L.

Typically, the concentration of the composition used in the contacting step is between 0.5 to 7 g/L, preferably 5 to 7 g/L.

In one embodiment the contacting step is carried out at a temperature from room temperature to the boiling point of the solvent. In one embodiment the contacting step is carried out at a temperature from 25 to 90°C. In one embodiment the contacting step is carried out at a temperature from 35 to 70°C.

In one embodiment the contacting step is carried out at a pressure of 0.3 to 1.2 bar. In one embodiment the contacting step is carried out at a pressure of 0.9 to 1.15 bar.

In one embodiment the contacting step is carried out for a time of 30 seconds to 2 hours. In one embodiment the contacting step is carried out for a time of 1 minute to 1 hour. In one embodiment the contacting step is carried out for a time of 5 minute to 40 minutes.

In one embodiment, the contacting step is carried out at a pH of 2 to 12. Without wishing to be bound by theory, it is believed that the pH of the reaction may influence the size of the resultant metal nanoparticles. Preferably, the contacting step is carried out at a pH of 5 to 8.

In one embodiment the contacting step is carried out under stirring. In one embodiment the stirring is carried out at 200 to 1000 rpm.

In one embodiment, a seed solution of metal nanoparticles (typically produced using the method of the present invention) is added to the solution of metal ions before the composition is added in the contacting step. Without wishing to be bound by theory, it is believed that adding the seed solution of metal nanoparticles may better control the growth of the nanoparticles. Typically, a volume of between 500nL and 1mL of seed solution is added before the contacting step.

In one embodiment the contacting step is followed by quenching of the reaction mixture. This can be carried out by any means known in the art. Typically this is carried out by cooling, for example using a jacketed reactor which allows heat transfer around the reactor without direct contact with the product solution.

After the contacting step, the reaction mixture is worked up in order to purify the metal nanoparticle. This can be carried out by methods known to those skilled in the art.

In one embodiment the product is purified by ion exchange chromatography. As is known to the person skilled in the art, ion-exchange chromatography separates molecules based on their respective charged groups by passing them through a matrix (typically a column) capable of ion exchange (typically an ion-exchange resin). Ion-exchange chromatography retains analyte molecules on the column based on coulombic (ionic) interactions. The ion exchange chromatography matrix consists of positively and negatively charged ions. Essentially, molecules undergo electrostatic interactions with opposite charges on the stationary phase matrix. The stationary phase consists of an immobile matrix that contains charged ionizable functional groups or ligand.

In one embodiment the product is purified by crystallisation. As is known to the person skilled in the art, this typically comprises forming a crystalline solid phase from a solution, typically from a supersaturated solvent.

In one embodiment part of the output from the crystallisation step is recycled to the input into the contacting step. It has been found that including a recycle loop around the contacting step minimises waste production throughout the process in order to maximise process conversion at the point where the metal ions are introduced, thereby avoiding the discharge of large amounts of metal ions into the environment.

In one embodiment the product is purified by centrifugation. The centrifugation may be carried out in a single centrifugation step or in multiple centrifugation steps (such as 2, 3, 4 or 5 steps). When the centrifugation is carried out in multiple centrifugation steps, the rate of centrifugation in each step, and the time for which the centrifugation is carried out in each step, may be the same or different.

In one embodiment, the centrifugation is carried out at 500 to 100,000 rpm. In one embodiment, the centrifugation is carried out at 500 to 16,000 rpm. In one embodiment, the centrifugation is carried out at 100 to 5000 rpm. In one embodiment, the centrifugation is carried out at 2000 to 3000 rpm. In one embodiment, the centrifugation is carried out at 1000 rpm. In one embodiment, the centrifugation is carried out at 2500 rpm. In one embodiment, the centrifugation is carried out at 5000 rpm. In one embodiment, the centrifugation is carried out at 7000 rpm.

Differential centrifugation can be used to tune nanoparticle size as a function of centrifugation speed. Multiple rounds of centrifugation can be used to increase metal nanoparticle purity.

In one embodiment, the centrifugation is carried out in two steps. In one embodiment, the first centrifugation step is carried out at 500 to 100,000 rpm, and the second centrifugation step is carried out at 500 to 100,000 rpm. In one embodiment, the first centrifugation step is carried out at 500 to 2000 rpm, and the second centrifugation step is carried out at 5000 to 20,000 rpm. In one embodiment, the first centrifugation step is carried out at 5000 to 20,000 rpm, and the second centrifugation step is carried out at 500 to 2000 rpm. In one embodiment, the first centrifugation step is carried out at 1000 rpm, and the second centrifugation step is carried out at 10,000 rpm. In one embodiment, the first centrifugation step is carried out at 10,000 rpm, and the second centrifugation step is carried out at 1000 rpm.

In one embodiment, the centrifugation is carried out for 20 seconds to 2 hours. In one embodiment, the centrifugation is carried out for 1 minute to 1 hour.

In one embodiment, the centrifugation is carried out for 10 to 30 minutes. In one embodiment, the centrifugation is carried out for 20 minutes. In one embodiment, the centrifugation is carried out for 2 minutes. In one embodiment, the centrifugation is carried out for 5 minutes. In one embodiment, the centrifugation is carried out for 10 minutes.

In one embodiment the product is purified by ultrasonication. In one embodiment, the ultrasonication is carried out at 30 to 50 kHz. In one embodiment, the ultrasonication is carried out at 35 to 45 kHz. In one embodiment, the ultrasonication is carried out at 0 to 50°C. In one embodiment, the ultrasonication is carried out at 15 to 40°C. In one embodiment, the ultrasonication is carried out for 1 minute to 30 minutes. In one embodiment, the ultrasonication is carried out for 10 to 20 minutes. In one embodiment, the ultrasonication is carried out for 15 minutes.

In one embodiment, the ultrasonication is carried out in two steps. The frequency, temperature and time of each step follows the general ultrasonication procedures described above. In one embodiment, the first ultrasonication step is carried out for 2 minutes to 15 minutes, and the second ultrasonication step is carried out for 1 minute to 10 minutes. In one embodiment, the first ultrasonication step is carried out for 10 minutes, and the second ultrasonication step is carried out for 5 minutes.

In one embodiment, the method comprises a shaking step following the or each ultrasonication step. In one embodiment, the shaking is carried out for 10 seconds to 30 minutes. In one embodiment, the shaking is carried out for 30 seconds to 15 minutes.

In one embodiment, the method comprises two ultrasonication steps, each followed by a shaking step. The conditions of each ultrasonication step and each shaking step follows the general ultrasonication and shaking procedures described above.

In one embodiment, the method comprises a step of increasing the pH in between the centrifugation and ultrasonication steps. Typically the pH is increased to between 10 and 14. Any suitable base may be used to carry this out provided it has no effect on the nanoparticles. Examples include alkali metal hydroxides, such as sodium hydroxide.

In one embodiment the product is purified by micro-, ultra- and/or nanofiltration. As is known to the person skilled in the art, microfiltration is a membrane filtration-based method that uses a membrane having micrometre-sized pores to filter particulate material. In addition, as is known to the person skilled in the art, ultrafiltration is a variety of membrane filtration in which forces, such as pressure or concentration gradients, lead to a separation through a semipermeable membrane. Also, as is known to the person skilled in the art, nanofiltration is a membrane filtration-based method that uses nanometre sized through-pores that pass through the membrane. Microfiltration membranes typically have pore sizes from 1-100 $\mu$m, and nanofiltration membranes typically have pore sizes from 1-100 nm, such as 1-10 nm. In one embodiment the product is purified by microfiltration. In one embodiment the product is purified by microfiltration followed by ultrafiltration. In one embodiment the membrane has a pore size between 1 nm and 1 $\mu$m. In one embodiment the membrane has a pore size between 100 nm and 500 nm.

In one embodiment, the method comprises a step of adjusting the pH after the ultrasonication step. Typically the pH is adjusted to between 7 and 9. Any suitable acid may be used to carry this out provided it has no effect on the nanoparticles. Weak acids, in particular organic acids are preferred, and acetic acid is especially preferred.

In one embodiment, the method comprises a step of drying the nanoparticles. In one embodiment, the drying step is carried out by vacuum drying. In a preferred embodiment, the drying step is carried out by vacuum distillation.

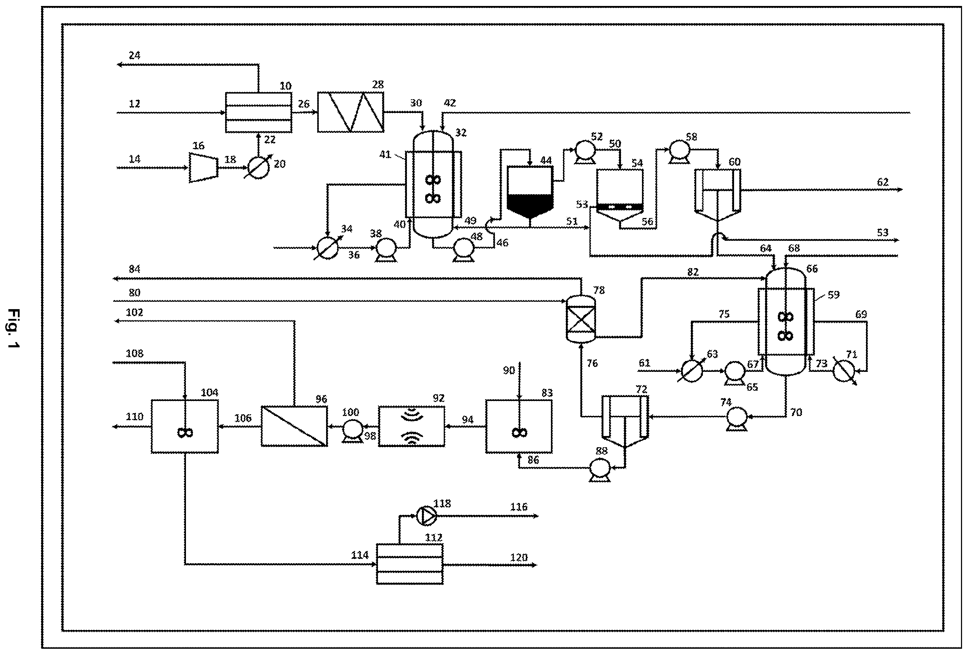

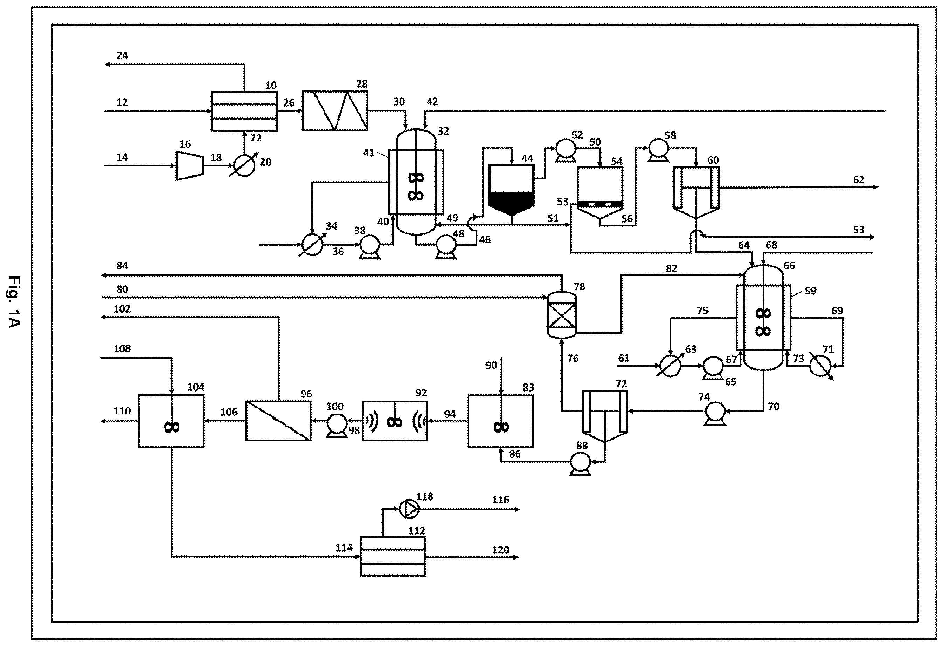

The preferred process is illustrated in more detail in Fig. 1. A solution of plants mixed in the appropriate ratio enters the drying unit 10 via line 12. Air is pumped via line 14, into compressor 16 and then via line 18 into heater 20. The heated air is then fed via line 22 into drying unit 10, exiting the drying unit via line 24.

Dried plant exits the drying unit 10 and is fed into blender 28 via line 26. The blended plant solution exits the blender and is fed into leaching unit 32 via line 30. Water is heated in heater 34, exits the heater via line 36 and is pumped via pump 38 and line 40 into a heating jacket 41 around leaching unit 32. Deionized water is introduced into the top of leaching unit 32 via line 42.

The solution exits the bottom of leaching unit 32 and is pumped via pump 48 and line 46 into decanter unit 44, in which it is to form a separated blend solution and a blend sludge. Some sludge exiting the decantation unit and is recycled back into leaching unit 32 via line 49. The blend sludge is removed via line 51.

The decanted solution of blended plants is fed from decantation unit 44 via line 50 and pump 52 into filtration unit 54. Further sludge is removed via line 53 into line 51. The solution exits filtration unit 54 via line 56 and pump 58 into centrifuge 60, which separates the solution and removed any further solid matter. Pellet waste exits centrifuge unit 60 via line 62.

The solution of blended plants exits centrifuge 60 via line 64 into reactor 66. A solution of metal ions is introduced via line 68 to the top of reactor 66, in which the biological reducing agents in the solution of blended plants react with the metal ions to form metal nanoparticles.

Water is introduced via line 61 into heater 63. The heated water is fed into a heating jacket 59 around reactor 66 via pump 65 and line 67. Water exits reactor 66 via line 69 into cooler 71 and is recycled back into reactor 66 via line 73. Water also exits jacket 59 via line 75 into heater 63 and is recycled back into jacket 59 via pump 65 and line 67.

The metal nanoparticles exit reactor 66 and are fed into centrifuge 72 via line 70 and pump 74. In centrifuge 72, the reaction mixture is separated into the nanoparticles and a solution of metal ions.

The metal ion solution exits centrifuge 72 and is fed into ion exchange chromatography unit 78 via line 76. Acid solution is fed into the unit 78 via line 80 to regenerate the ion exchange resin. The purified metal ions exit ion exchange chromatography unit 78 and are recycled back into reactor 66 via line 82. Waste solution exits the ion exchange chromatography unit 78 and is removed via line 84.

The metal nanoparticles exit centrifuge 72 and are fed into mixer 83 via line 86 and pump 88. A base (preferably sodium hydroxide solution) is introduced into mixer 83 via line 90. The pH is adjusted in mixer 83, and the metal nanoparticles exit mixer 83 and are fed into ultrasonicator 92 via line 94.

Following ultrasonication, the metal nanoparticles exit ultrasonicator 92 and are fed into membrane 96 via line 98 and pump 100. Nanoparticles of excess size (typically greater than 200 nm) are removed by the membrane 96 and exit via line 102.

The metal nanoparticles which pass through the membrane 96 are fed into mixer 104 via line 106. Acid solution is introduced into mixer 104 via line 108 to adjust the pH. Colloidal nanoparticles are removed from the mixer 104 via line 110.

The metal nanoparticles exit mixer 104 and are fed into dryer 112 via line 114. Water is removed in dryer 112 and exits dryer 112 via line 116 and pump 118. The finished nanoparticle powder exits dryer 112 via line 120.

An alternative process is illustrated in Fig. 1A. This process embodiment differs from that of Fig. 1 in that unit 92 is a hybrid unit in which both ultrasonication and mixing take place.

**Applications** - The following embodiments are not according to the invention and are present for illustration purposes only

The metal nanoparticles may be incorporated into a wide variety of products and methods.

Therefore, disclosed herein is a product including the metal nanoparticle.

In one embodiment, disclosed herein is a cosmetic product including the metal nanoparticle.

In one embodiment, disclosed herein is a battery including the metal nanoparticle.

In one embodiment, disclosed herein is a pharmaceutical product (which may be an active pharmaceutical ingredient, a pharmaceutical excipient or a drug delivery agent) including the metal nanoparticle.

In one embodiment, disclosed herein is a plant protection product, such as an agricultural chemical product (which may be an active agrochemical ingredient, an agrochemical excipient or an agrochemical delivery agent) including the metal nanoparticle.

In one embodiment, disclosed herein is a construction material including the metal nanoparticle.

In one embodiment, disclosed herein is a detergent or cleaning product including the metal nanoparticle.

In one embodiment, disclosed herein is a textile product or article of clothing, an article of footwear, or an accessory (such as a jewellery item, a bag, or other fashion item) produced therefrom including the metal nanoparticle.

In one embodiment, disclosed herein is a biological sensor including the metal nanoparticle.

In one embodiment, disclosed herein is a lubricant including the metal nanoparticle.

In one embodiment, disclosed herein is an optic or optoelectronic device including the metal nanoparticle.

In one embodiment, disclosed herein is a conductive ink including the metal nanoparticle.

In one embodiment, disclosed herein is an antimicrobial agent including the metal nanoparticle.

In one embodiment, disclosed herein is a biocidal agent (including a pesticide) including the metal nanoparticle.

In one embodiment, disclosed herein is a catalyst including the metal nanoparticle.

In one embodiment, disclosed herein is an enhanced material (such as a polymer, which may be a nanoparticle-embedded polymers, or a functionalised material or derivative, including the metal nanoparticle).

In one embodiment, disclosed herein is a preservative (such as a food preservative) including the metal nanoparticle.

In one embodiment, disclosed herein is a filter (for example, a filter for air and/or water purification) including the metal nanoparticle.

In one embodiment, disclosed herein is a machine (such as a robot, which may be a nano-robot) including the metal nanoparticle.

In one embodiment, disclosed herein is the use of the metal nanoparticle in biomedical imaging.

In one embodiment, disclosed herein is the use of the metal nanoparticle in gene sequencing.

In one embodiment, disclosed herein is the use of the metal nanoparticle in information storage and processing (including nanocomputing and quantum computing applications).

In one embodiment, disclosed herein is the use of the metal nanoparticle in nanoelectronics.

## Examples

### General

UV/VIS spectra were recorded on a Visible Spectrophotometer (721 LDC Digital Lab Spectrophotometer). Scanning electron microscopy (SEM) measurements were carried out using a field emission SEM from Hitachi, type SU8230 and SU8200. Energy Dispersive X-Ray Spectroscopy (EDX) spectra were recorded on SEM from Hitachi, type SU8230 and SU8200 using a detector from Bruker, type XFlash.

### Reference Example 1- development of optimal blends by GAMS Modelling

Blend optimal compositions were obtained by a multi-objective linear optimisation program using the ε-constraint method resulting in the construction of pareto frontiers in GAMS. The two variables being optimised were the antioxidant capacity per 100g and the cost per 100g.

Antioxidant capacity was used as a pro-environment variable. If the antioxidant capacity for the same mass of blend is higher, then less plants would be required for the bio-synthesis of metallic nanoparticles, resulting in a reduction in material and energy usage. As outlined in the article "Design Through the 12 principles of Green Engineering" (Paul T. Anastas et al., *Environ. Sci. Technol.* **2003,** 37(5), 94A-101A) this would result in environmental benefits from less direct and indirect resource consumptions, from $CO_2$ and $SO_x$ emissions reduction in energy production as well as less water to grow the plants. Therefore, this would decrease upstream environmental impacts. The process has been evaluated as a whole and its impacts on the environment under a life cycle analysis framework. As lower optimisation constraints, water, sugar, zinc, iron and carbohydrates content per 100g, and as upper constraints Vitamin C (ascorbic acid), lutein and zeaxanthin and carotene content per 100g were used. The amount of water in the blend was minimised in order to decrease downstream energy costs since the first step in the production process is drying. In view of the high enthalpy of vaporisation of water (40.65 kJ/mol) this resulted in a significant energy requirement decrease upon process scale-up.

It was also possible to decrease the amount of sugar and carbohydrates in order to inhibit potential microbial growth in the blends allowing them to be conserved for a longer period of time. Zinc and iron amounts were minimised in order to avoid other metallic nanoparticles (MNPS) formation as well as disturbances in the zeta potential post-production of the nanoparticles, which could result in the agglomeration of the MNPS rendering them not fit for use. Finally, water-soluble antioxidants (ascorbic acid, xanthophylls) as well as fat-soluble antioxidants (carotenes) were sensibly maximised taking into consideration economic cost constraints.

The base case constraint developed is set as outlined in Table 1.

Table 1

| Constraints | Quantity (g/100g) |
|---|---|
| Water | 85 |
| Sugars | 1.5 |

(continued)

| Constraints | Quantity (g/100g) |
|---|---|
| Iron | 0.01 |
| Zinc | 0.001 |
| Carbohydrates | 20 |
| Ascorbic Acid | 0.02 |
| Carotenes | 0.003 |
| Lutein + Zeaxanthin | 0.001 |

Plants and spices readily available in the UK were included in the blending model: Artichoke, Curly Kale, Red Cabbage, Oregano, Rosemary, Sage and Watercress. Their nutritional composition was obtained from various online databases of which myfooddata.com being the main one. Since a linear program was used, all optima (in this case minima) are considered being global optima if they belong to the feasible set. Cost per 100g was based on local supermarkets pricing. The results are shown in Table 2.

Table 2

| Composition | Antioxidants (mmol/100g) | Cost (£/100g) | Antioxidant Ratio (BX/WC) | Cost Decrease (%) |
|---|---|---|---|---|
| Watercress | 2.63 | 1.42 | 1 | N/a |
| Blend 1 | 7 | 0.501 | 2.66 | 65% |
| Blend 2 | 10 | 0.532 | 3.80 | 63% |
| Blend 3 | 12 | 0.586 | 4.56 | 59% |
| Blend 4 | 15 | 0.666 | 5.70 | 53% |
| Blend 5 | 17 | 0.719 | 6.46 | 49% |
| Blend 6 | 19 | 0.886 | 7.22 | 38% |
| Blend 7 | 20 | 1.02 | 7.60 | 28% |
| Blend 8 | 21 | 1.15 | 7.98 | 19% |
| Blend 9 | 22 | 1.486 | 8.37 | -5% |

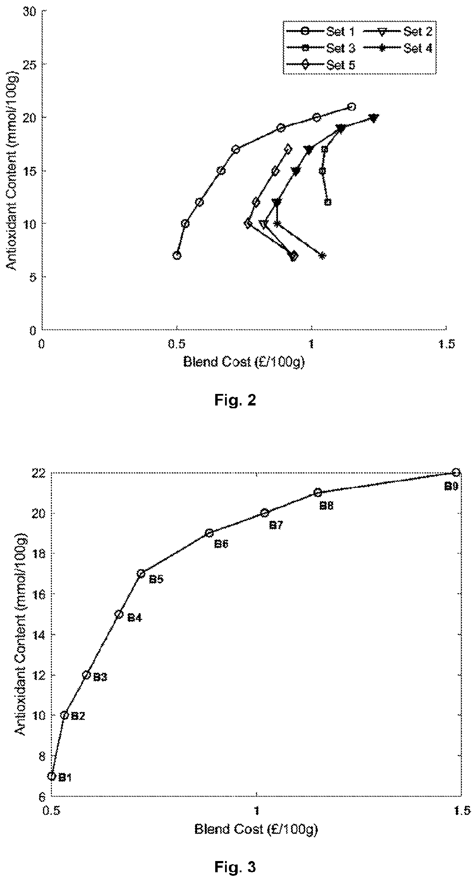

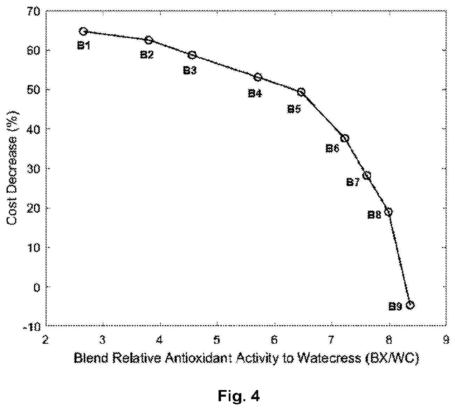

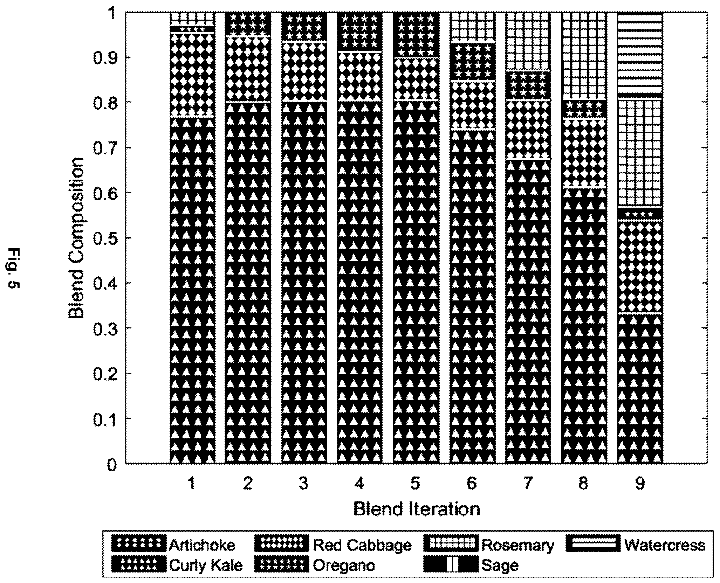

Finally, 9 blends were obtained using the programming software, which allowed the construction of a Pareto Frontier, as described on Figure 3. As it can be seen on Figure 5, the main component of each blend is curly kale, with either artichoke or red cabbage being used as "fillers" and the spices; oregano, sage, rosemary, are being used as rich sources of antioxidants. The antioxidants have been used to reduce metallic ions into nanoparticles. It can be noted that as more the antioxidant capacity of the blend increases the more the cost per 100g does. Finally, from figure 4 and table 2 one can understand that nearly all the blends to the exception of blend 9 are superior to the only watercress solution which has been extensively used in previous art (under the economic point of view). This is due to blend 9 containing an equal distribution of each plant instead of the preferred [main-filler-rich] configuration. Therefore, it was recommended to test blends which are both economically and chemically preferable for the reaction synthesis. However, it must be noted that an equal distribution of plant variety allows the highest antioxidant potential, therefore requiring less material and saving overall economic resources in processing.

**Reference Example 2- DPPH Assay - Experimental Verification & Synergetic Interaction**

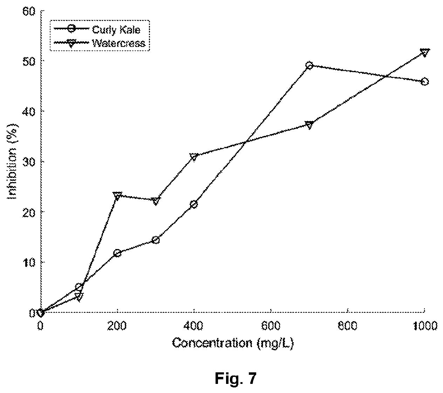

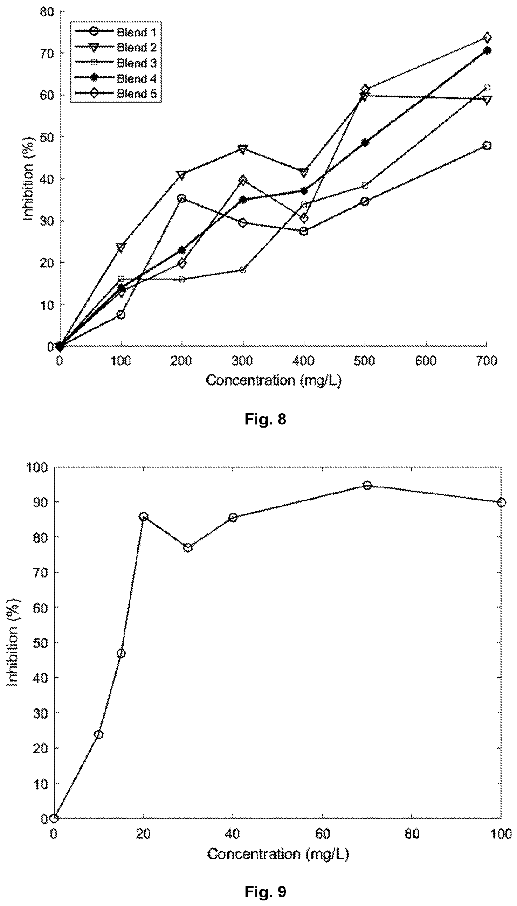

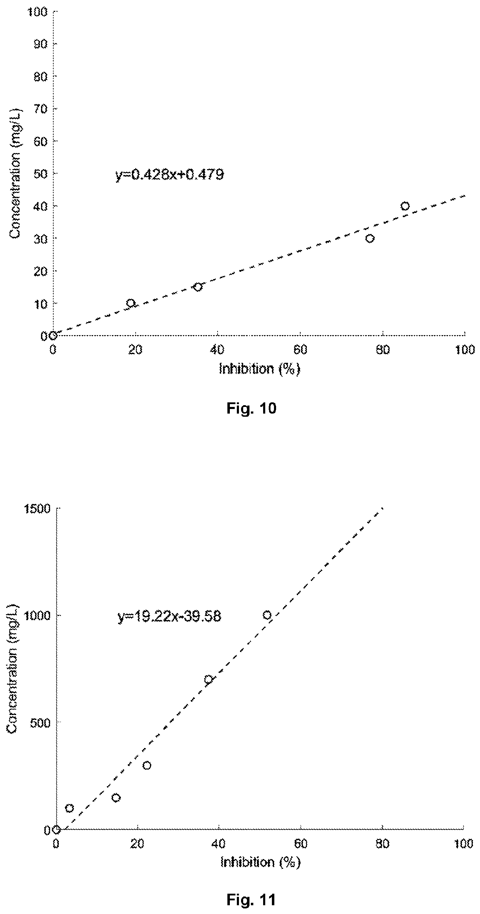

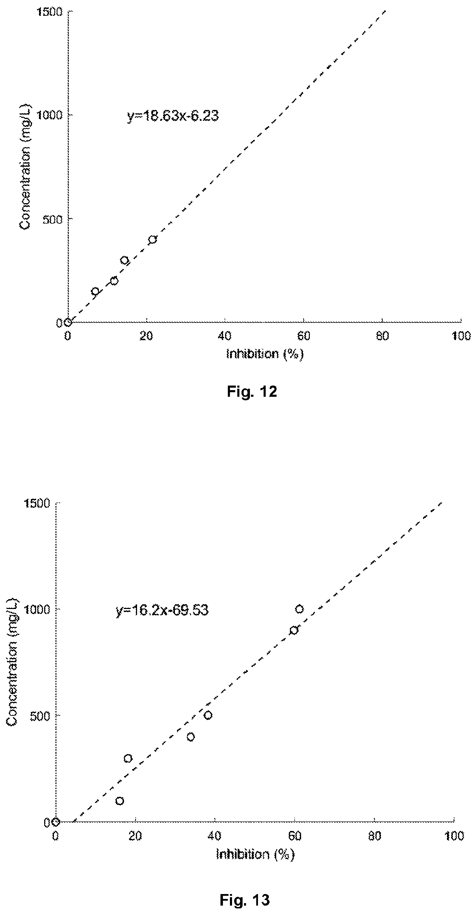

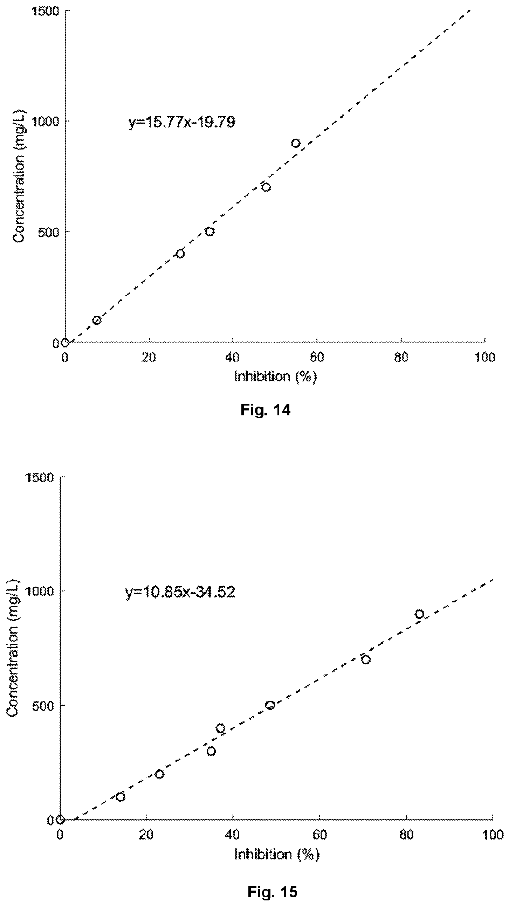

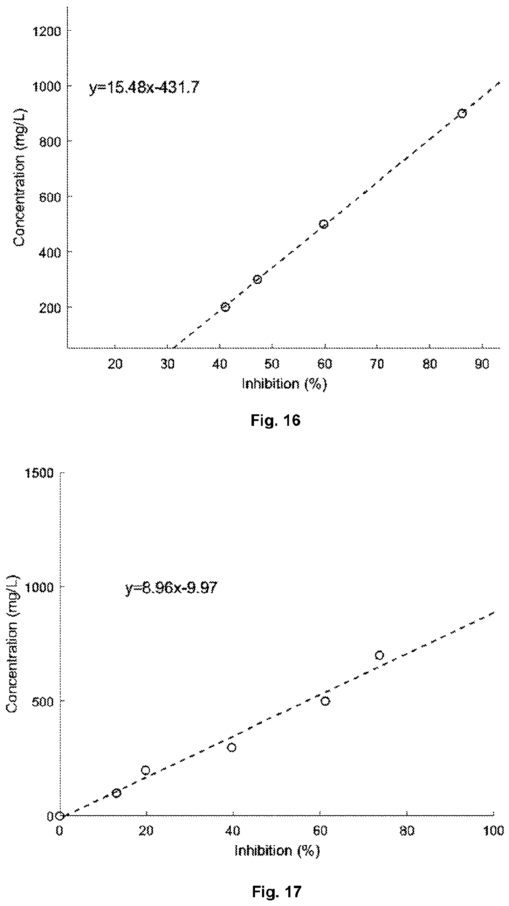

In order to validate the results obtained from the optimisation algorithm it was possible to experimentally verify the antioxidant content of the produced blends. To do so, individual plant extracts as well as blends extracts were tested and their relative strengths reducing a free radical, 2,2-diphenyl-1-picrylhydrazyl (DPPH) were evaluated. Pure ascorbic acid was used as a benchmark antioxidant. A colorimetric assay protocol using DPPH has been developed and was used to assess antioxidant activity. The corresponding 50% inhibition concentration of the free radical in solution was determined for each blend and individual plant. The 50% inhibition points ($IC_{50}$) were determined by linear regression which allowed the prediction of the $IC_{50}$ concentrations (Figure 7 to Figure 17). Every experiment was duplicated and averaged. The following equations were used for calculations:

$$\text{DPPH scavenging activity (\%)} = [(Ab - At) / Ab] \times 100$$

Ab: Blank Absorbance
At: Sample measured Absorbance

$$\text{Relative Strength} = IC_{50}(\text{sample})/IC_{50}(\text{ascorbic acid})$$

(can also be done relative to other antioxidants for instance watercress - WC)

Table 3

| Blend No | Theoretical $IC_{50}$ (mg/L) | Actual $IC_{50}$ (mg/L) | Variation towards Watercress (%) | Variation towards Theoretical (%) |
|---|---|---|---|---|
| 1 | 871 | 741 | 25% | 18% |
| 2 | 925 | 769 | 20% | 20% |
| 3 | 508 | 508 | 81% | 0% |
| 4 | 932 | 342 | 169% | 172% |
| 5 | 947 | 438 | 110% | 116% |

Table 4

| | Concentration (mg/L) | $IC_{50}(X)/IC_{50}(AA)$ |
|---|---|---|
| $IC_{50}$(Ascorbic Acid) | 21.9 | 1 |
| $IC_{50}$(Blend 4) | 342.3 | 15.7 |
| $IC_{50}$(Oregano) | 363.8 | 16.6 |
| $IC_{50}$(Blend 5) | 438.1 | 20 |
| $IC_{50}$(Blend 3) | 508.0 | 23.2 |
| $IC_{50}$(Sage) | 653.2 | 29.9 |
| $IC_{50}$(Blend 1) | 740.5 | 33.6 |
| $IC_{50}$(Rosemary) | 754.7 | 34.5 |
| $IC_{50}$(Blend 2) | 768.7 | 34.8 |
| $IC_{50}$(Artichoke) | 811.7 | 37.1 |
| $IC_{50}$(Watercress) | 921.2 | 42.1 |
| $IC_{50}$(Curly Kale) | 925.3 | 42.8 |
| $IC_{50}$(Red Cabbage) | 1294.6 | 59.2 |

From table 4, it is possible to observe that from the tested blends from figure 5, all the blends had a superior DPPH $IC_{50}$ than watercress. It is interesting to note that curly kale performs worst when alone, but is stronger when used as majority antioxidant source in a blend. All the blends resulted having higher relative strengths than most single components, with blend 4 (which does not use watercress) having a higher relative strength than oregano, the second highest single $IC_{50}$ concentration after ascorbic acid, even though oregano and rosemary are minority components.

This was unexpected. Therefore, it was decided to compute theoretical $IC_{50}$ concentrations and compare these to experimental concentrations. It can be noticed on table 3, that nearly every blend has a higher reducing capacity than that predicted using linear programming. This can be as much as over 150% stronger reducing capacity than what was theoretically predicted using mathematical modelling, as in the case of blend 4.

Therefore, in light of these results it was concluded that a synergistic underlying mechanism is present during the reduction of DPPH when blends are used compared to individual plants. This was surprising since under 5% deviations were expected from theoretical models. On top of the $IC_{50}$ variations, the fact that curly kale in these specific combinations, can exhibit a stronger reducing activity than when alone, even though remaining the major component in the blend, only can point to an enhancement and optimisation according to the present invention.

**Reference Example 3 - Method of producing the composition**

The selected plants were subsequently dried for 24 hrs at 40°C and 1 bar. The dried plants were then collected and blended in different ratios using a blender until particles of under 100 $\mu$m were obtained. After blending, the powder was recovered and leached in a tank by adding DI Water at 50°C and 1 bar for 20 minutes under continuous stirring. The stirring tank is connected to a decanter which itself forms three phases (Scum, Clarified Liquid, Sludge). The sludge is recycled back to the mixer for further processing to extract the maximum amount of antioxidants, until the concentration in the solid particles becomes minimal. At this point the depleted plant particles are disposed from the process. The clarified liquid is then recovered and filtered under pressure through various grades of filter membranes (microfiltration) in order to remove particles in suspension down to 1 $\mu$m. The filtrate is then differentially centrifuged (also at 2500 RPM for 10 minutes) allowing smaller particles to be discarded in the pellet.

**Example 4 - method of producing a silver metal nanoparticle**

An ionic solution is prepared at a desired concentration between 0.5 g/L to 1.5 g/L and is preheated in a reactor to 70°C for 1hr at 1 bar under continuous stirring. When a steady temperature value is achieved, a blend concentration between 0.5 g/L to 7 g/L is added to the reactor and nucleation instantaneously initiates.

Silver acetate ($AgCH_3COO$) was used as an alternative to silver nitrate ($AgNO_3$). This avoids the presence of $NO_3^-$ ions in solution which are toxic and bad for the environment. The crystallisation lasts between 5 minutes to 2 hours depending on the desired conversion and nanoparticle size. It can be noticed that nanoparticles grow as more time lapses, therefore suggesting the tuning of nanoparticle size with reaction time. This is when the bio-reduction occurs and the metallic nanoparticles grow through primary heterogeneous nucleation. A colour change from transparent to brown/black can be observed during this period.

The reaction is then quenched using cooling water at 4°C until the internal temperature of the mixture reaches 10°C. The mixture of nanoparticles and plant extract is subsequently recovered and differentially centrifuged (2500 RPM for 20 minutes). The supernatant is recycled back to the reactor in order to increase conversion. It passes through a cation exchange resin in order to recover a maximum amount of pure metal ion to be recycled back. Inactivated antioxidants in the process are purged. The pellet (with traces of water) is recovered and NaOH is added to increase the pH between 10 to 14. The pellet is then ultrasonicated at 40kHz at 25°C for 15 minutes.

Thereafter, the colloid is filtered through a micromembrane in order to remove any large agglomerates or deformed particles to ensure high quality functional nanoparticles with high surface area to volume ratios. This is an especially useful property for antimicrobial applications, conductive inks, and catalysis. Prior to shipping, the solution pH is adjusted to neutral or slightly alkaline (between pH=7 to pH=9) using a weak organic acid such as acetic acid in order for the solution not to be corrosive for users.

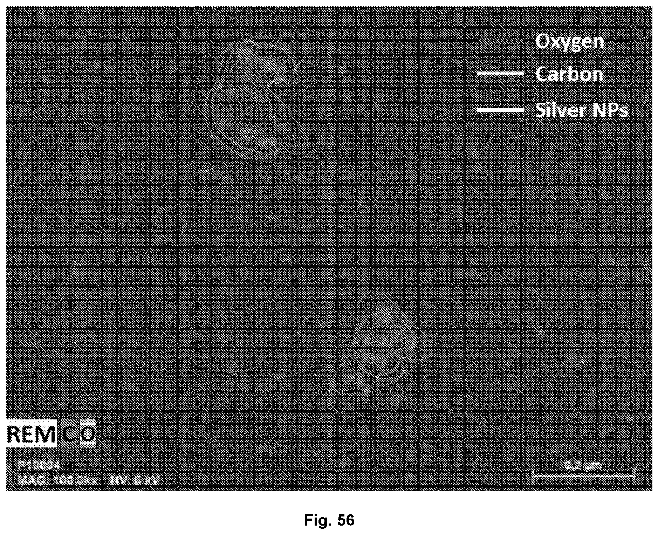

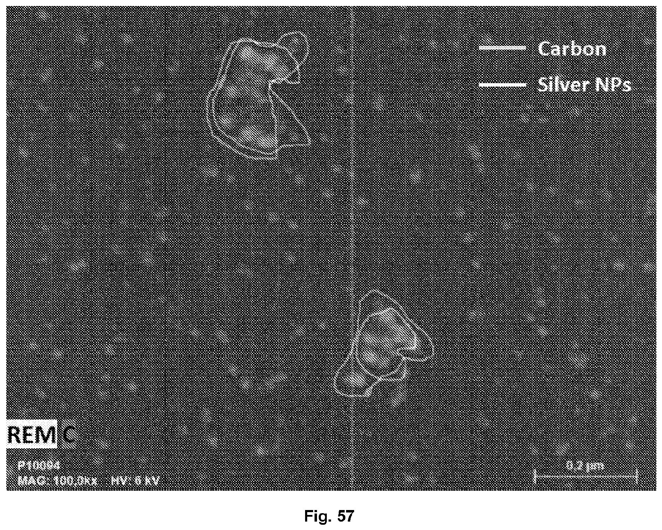

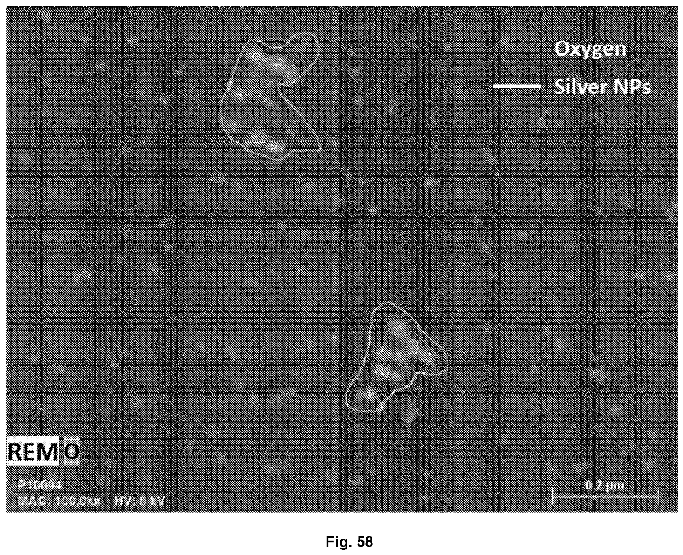

The colloid can also be sent to a vacuum dryer in which the water is removed at room temperature under vacuum (under 3000 Pa). This allows the formation of a nanopowder which then can be re-dissolved to produce other nano-enabled products such as conductive inks which require higher concentrations to become conductive. Silver nanoparticles are finally obtained in a colloidal solution. Naturally occurring bioagents in the extract served (supposedly including carbohydrates) as capping agents of the nanoparticles as it has been highlighted from Energy Dispersive X-Ray Spectroscopy results (see Figures 56-58).

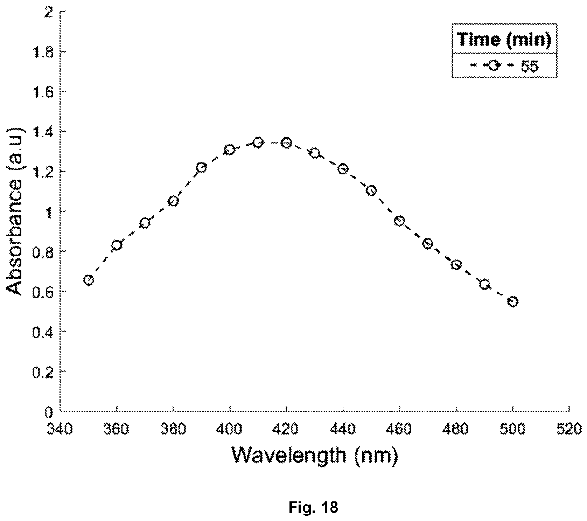

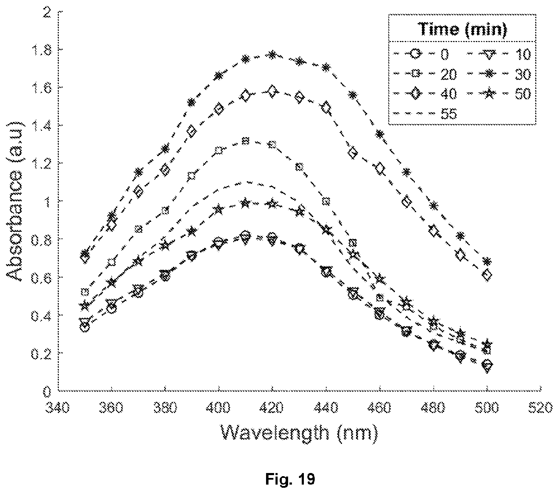

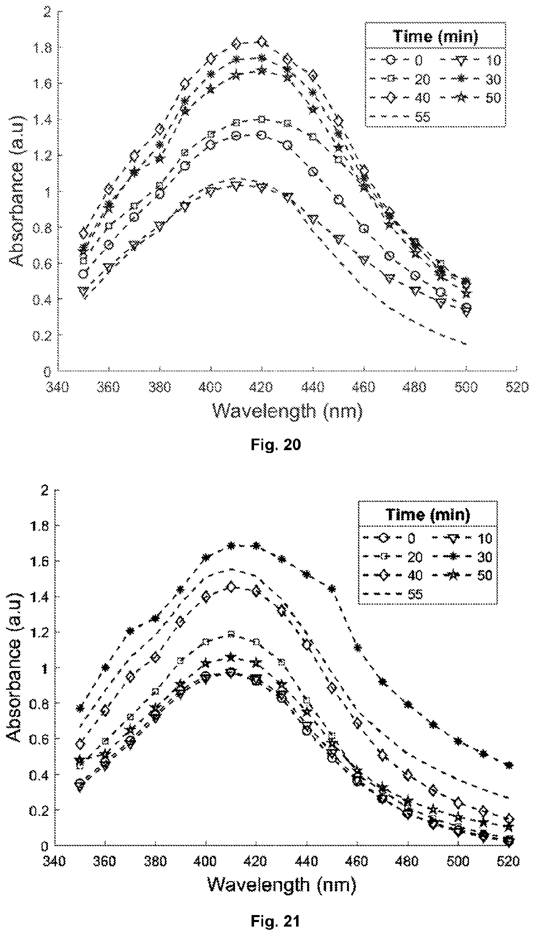

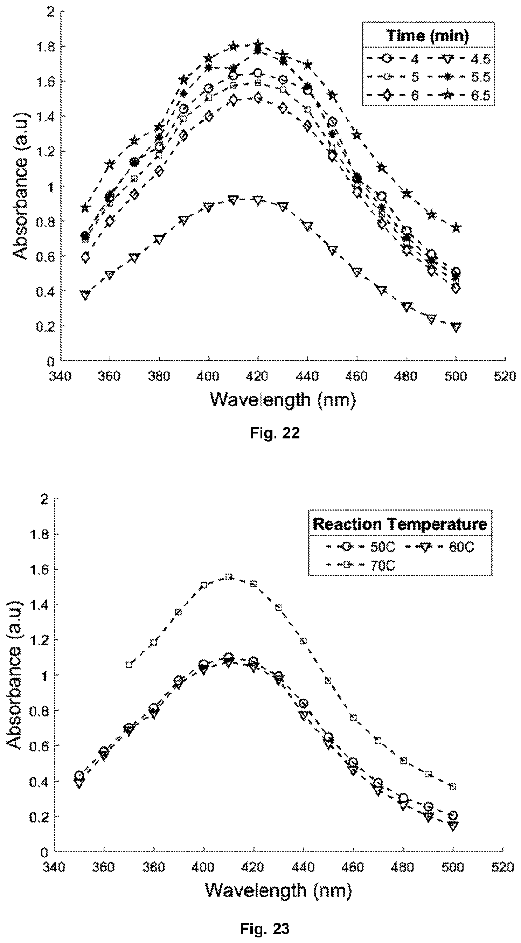

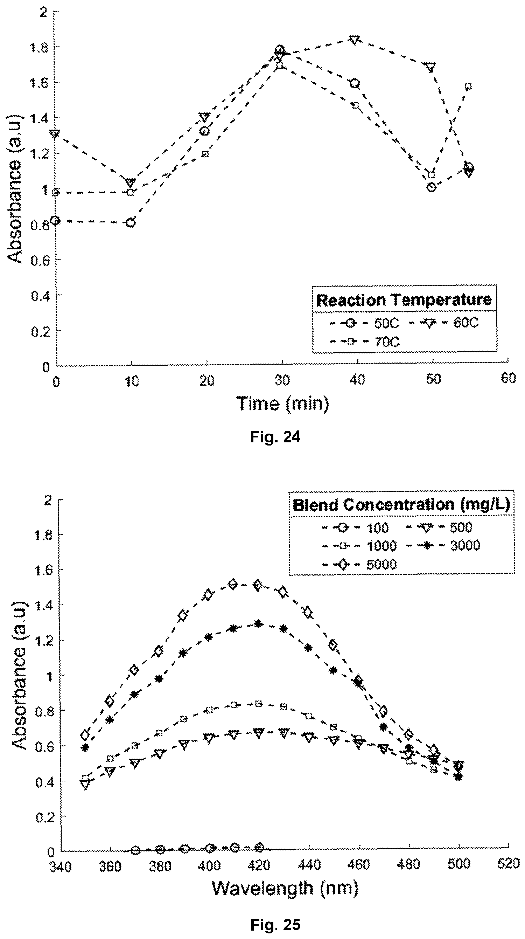

It was possible to verify that the chemical reaction is possible from room temperature onwards (25°C). Tests were carried out at 35°C, 40°C, 50°C, 60°C, 70°C and 85°C (Figures 18-22). Atmospheric pressure and above allow metallic nanoparticle production. Sufficient nanoparticles were produced in each case and were identified using spectral analysis (UV/VIS). It was also possible to test the effect of blend concentration on nanoparticles formation (Figure 25). It was possible to observe that 500 mg/L of blend was the threshold concentration for sufficient reduction of $Ag^+$ to occur and form nanoparticles.

On figure 24 it is possible to observe the cyclic nature of the crystallisation which follows the LaMer nucleation mechanism. After that nucleation occurs, the nanoparticles increase in size, which in turn increase nanoparticle concentration in solution until a critical concentration occurs. The critical concentration is achieved at 30 minutes after the reaction begins. After the concentration decreases until it reaches a stable value, which corresponds to a thermodynamic stability achieved by the colloidal solution explained by a complex balancing of attractive and repulsive forces acting on the nanoparticles (Gibbs free energy equilibrium is reached).

From UV-Vis analysis, it was possible to determine (by the method of Paramelle et al. Analyst, 2014, 139, 4855-4861) that the nanoparticles produced at the reactor exit had diameters between 38 - 50 nm.

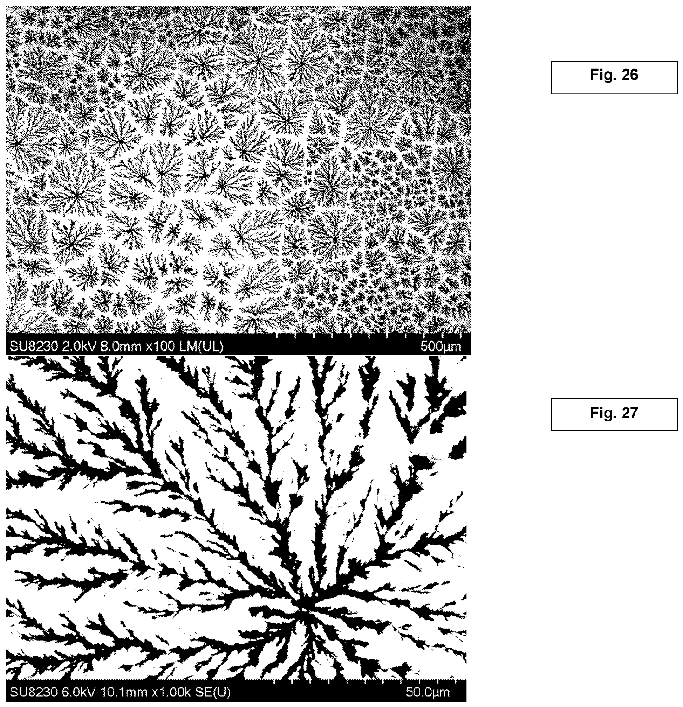

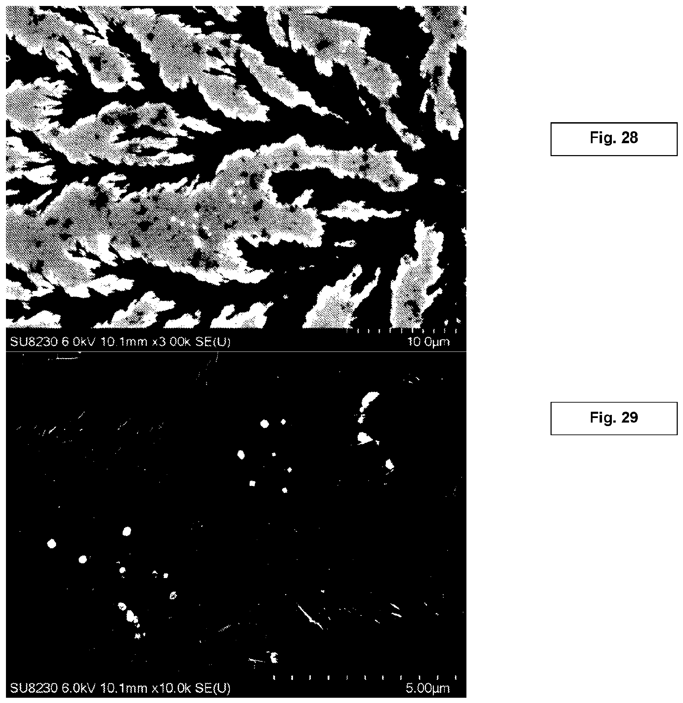

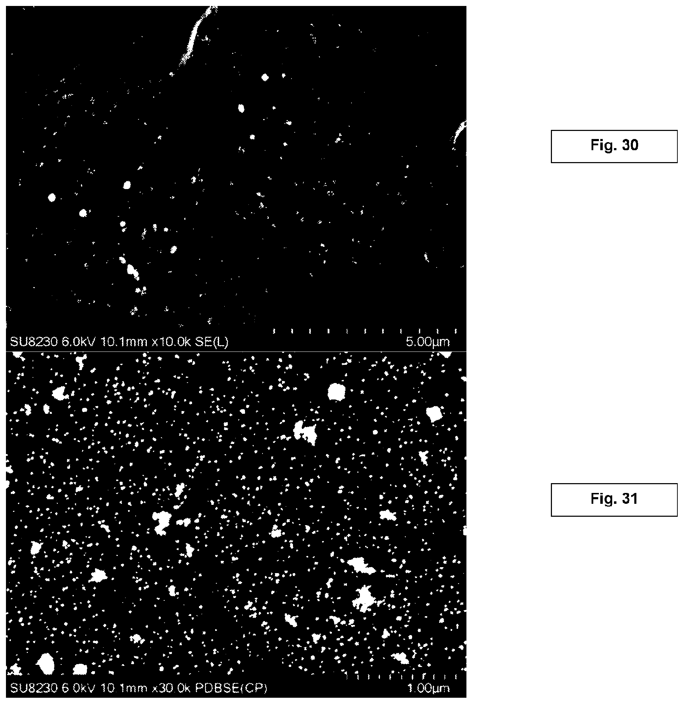

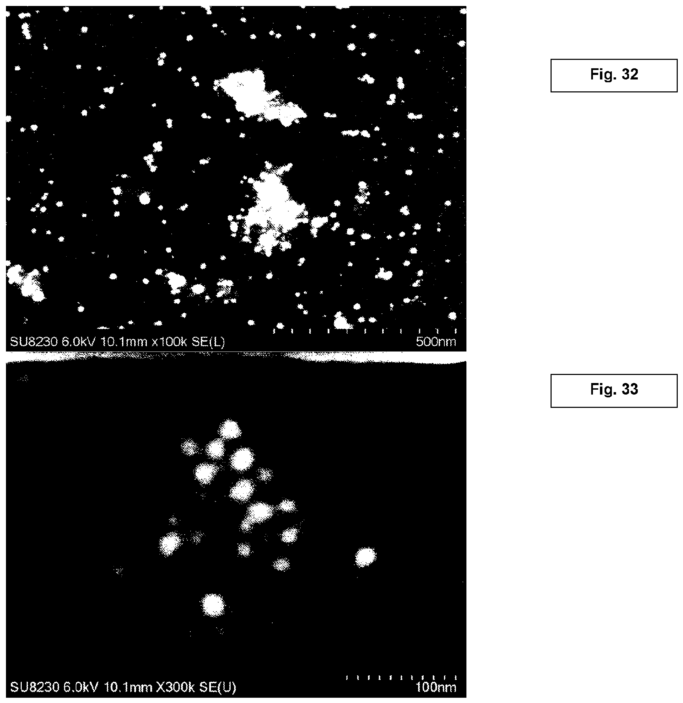

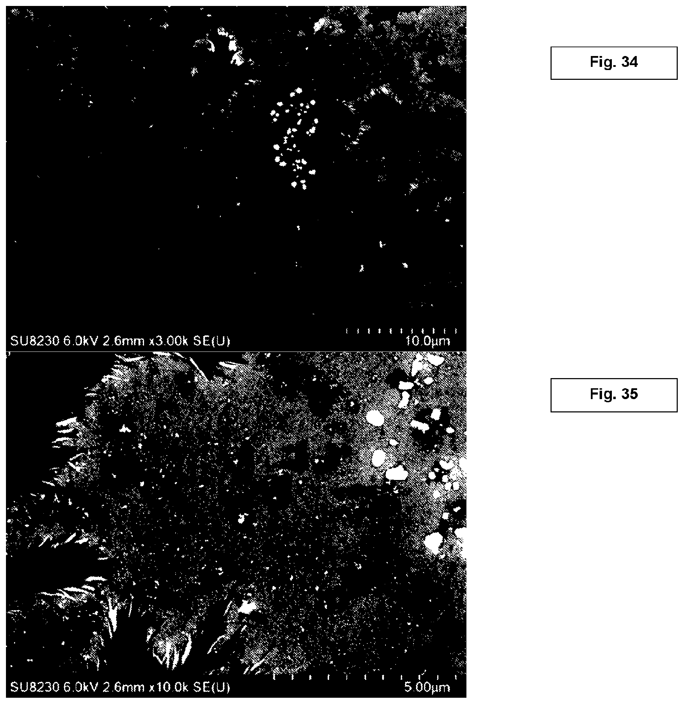

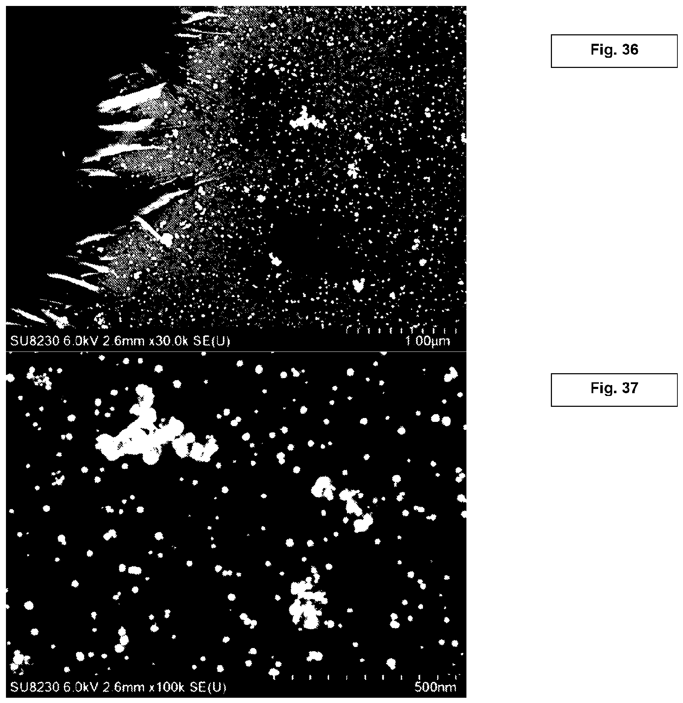

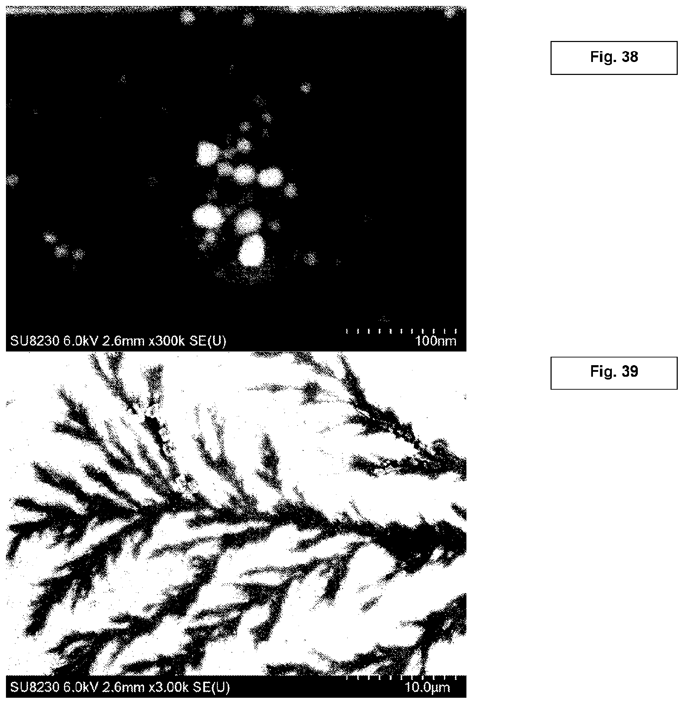

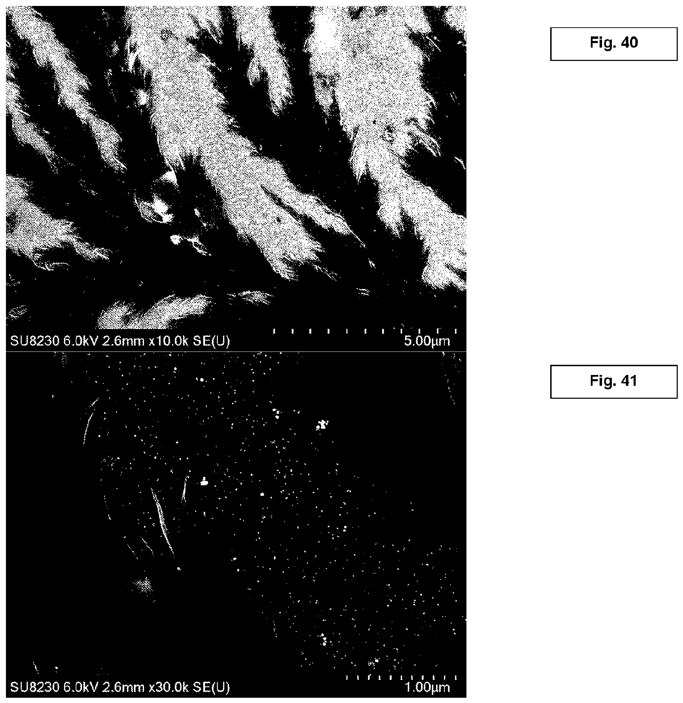

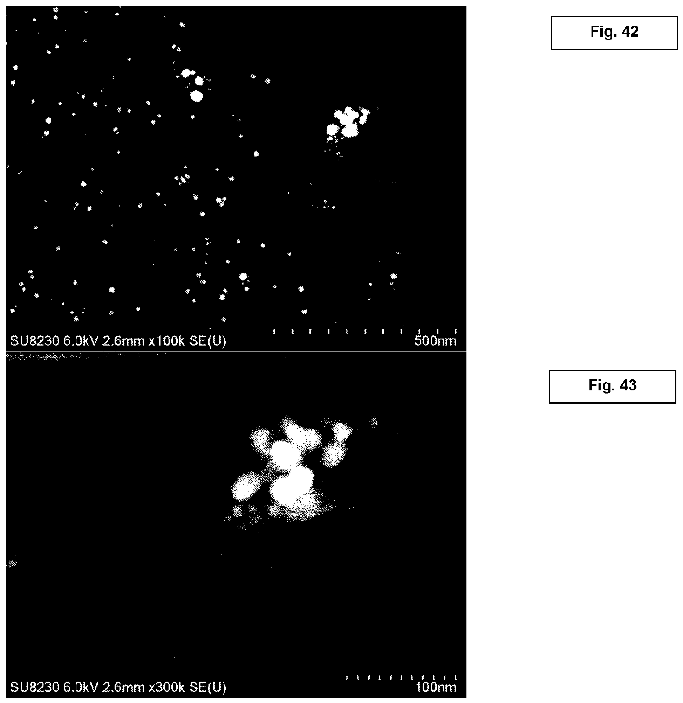

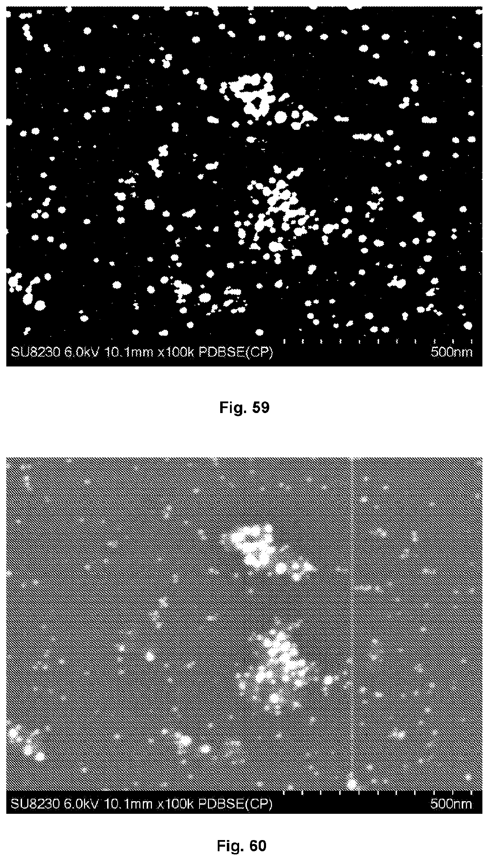

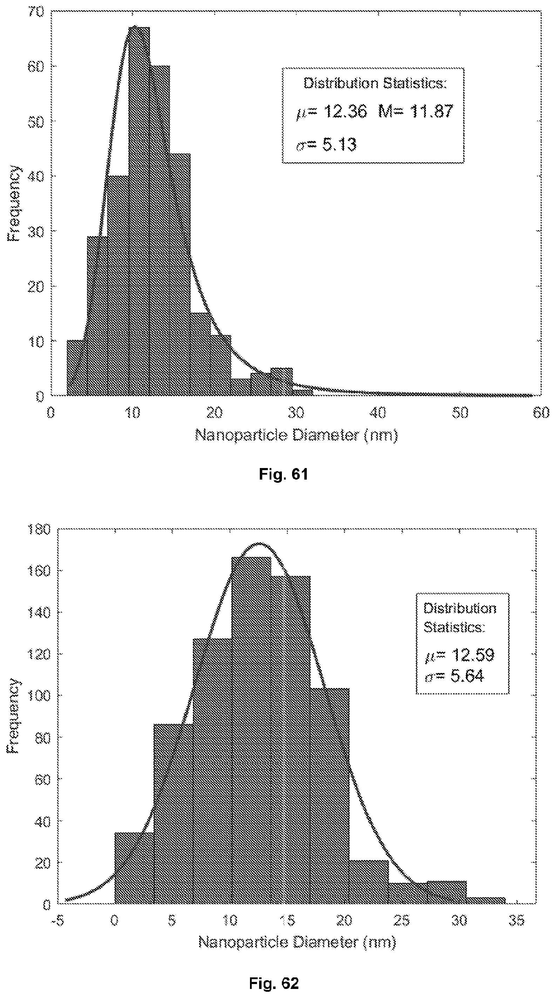

Post - reactor, the nanoparticles were filtered using microfiltration. 200nm membranes were used in order to discard any large agglomerate which may have remained post ultrasonication. Filtered samples were subjected to SEM and it was possible to observe nanoparticles of sizes between 5nm to 30nm as illustrated on figures 26 to 43 and figures 59 to 62. From figure 62, it is possible to observe that on a large sampling of nanoparticles a normal distribution is obtained which has an average diameter at 12.59 nm and a standard deviation of 5.64 nm. The nanoparticles are well distributed and non-agglomerated and had good sphericity (>70%).

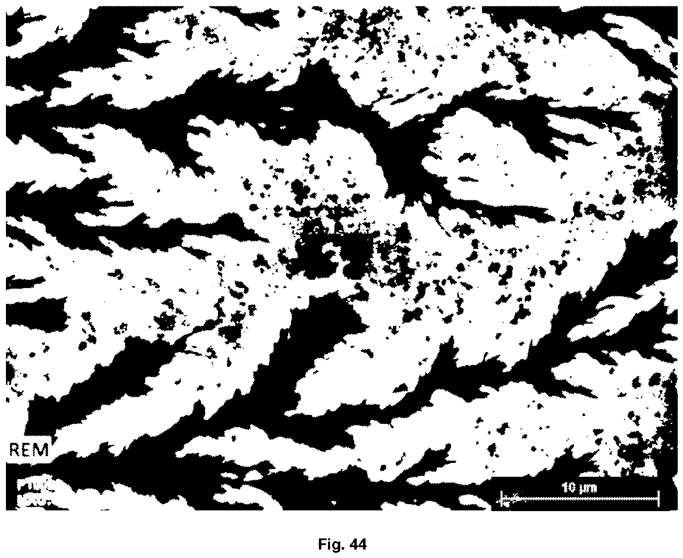

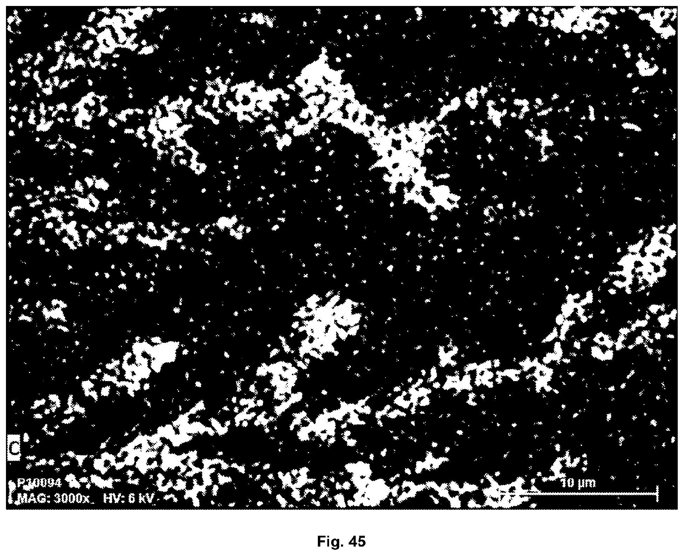

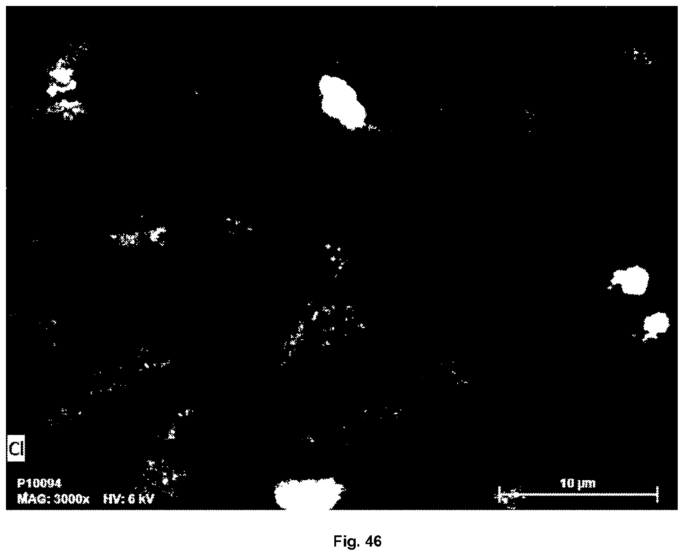

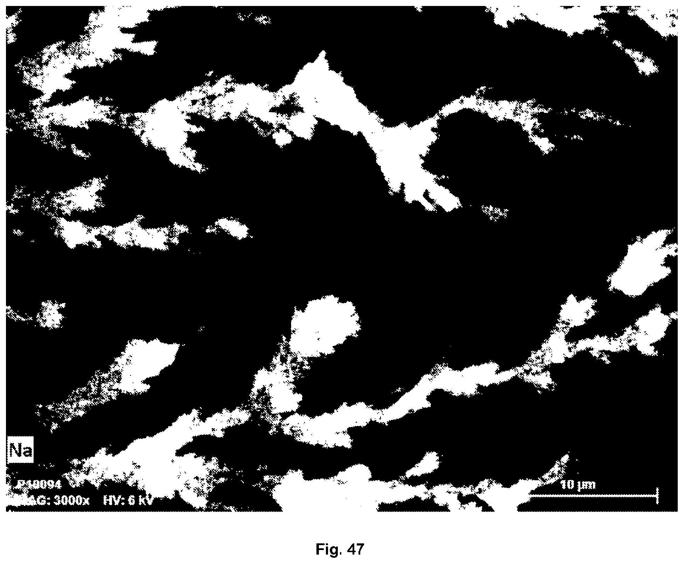

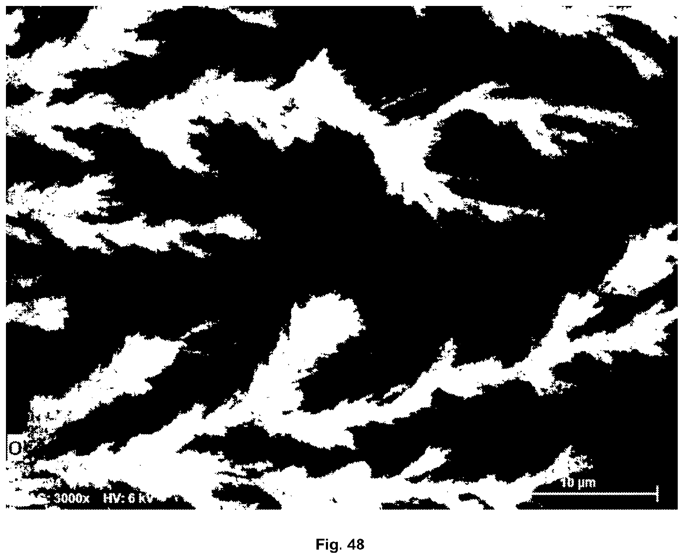

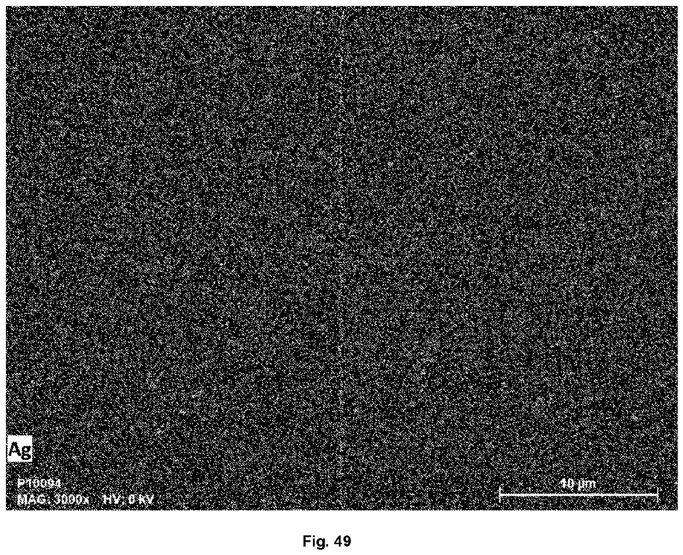

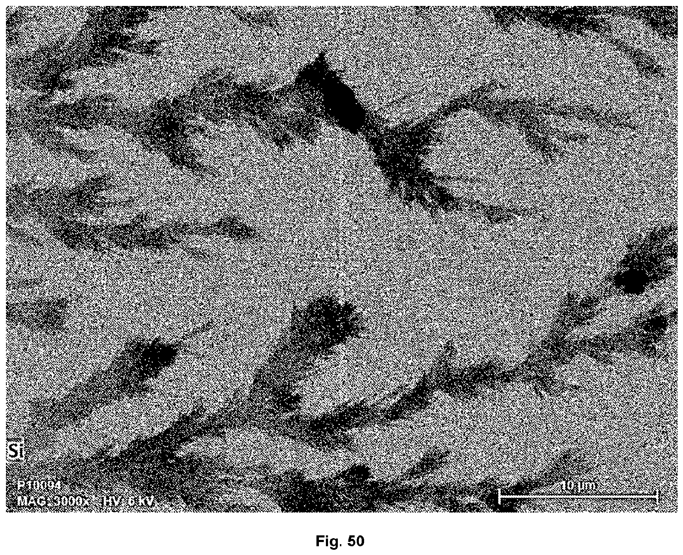

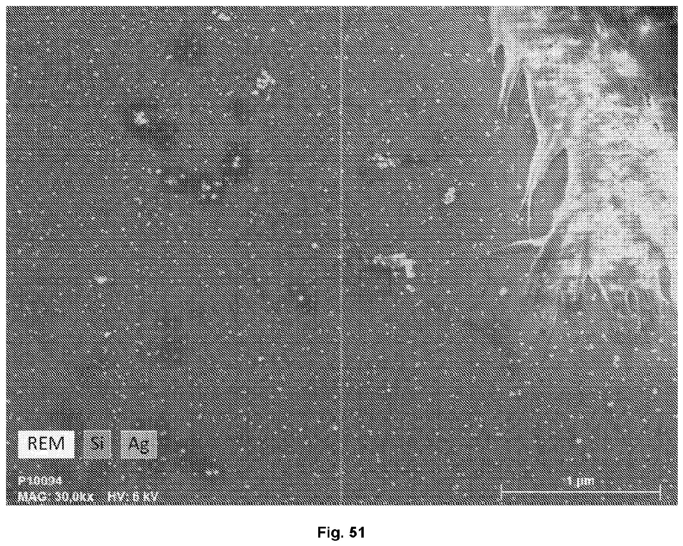

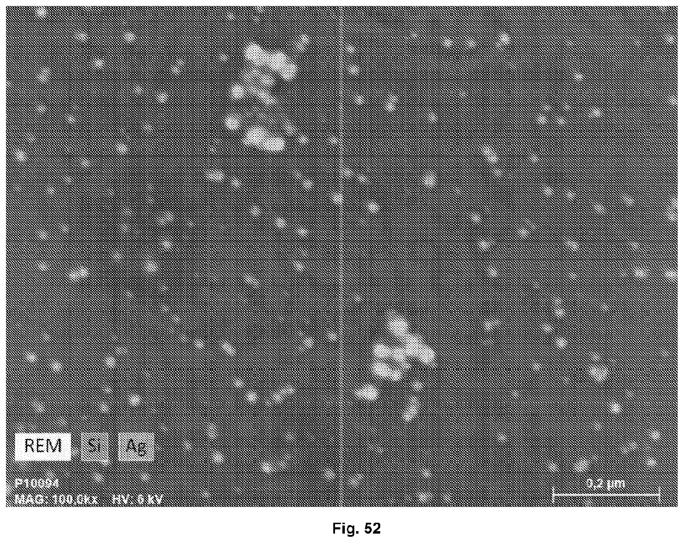

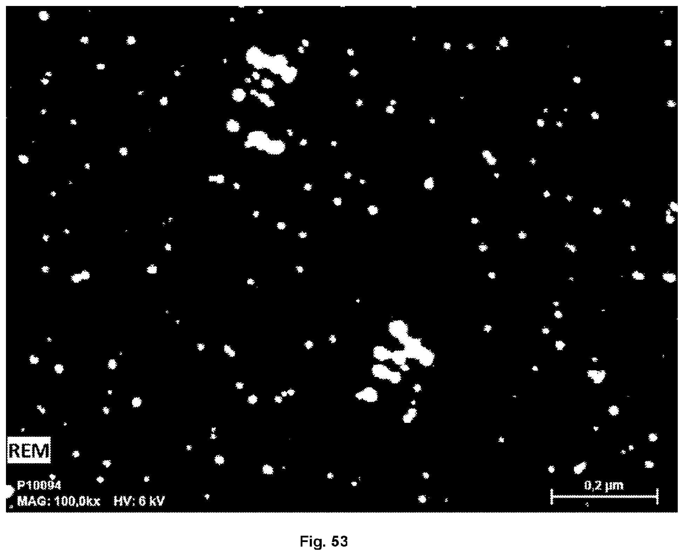

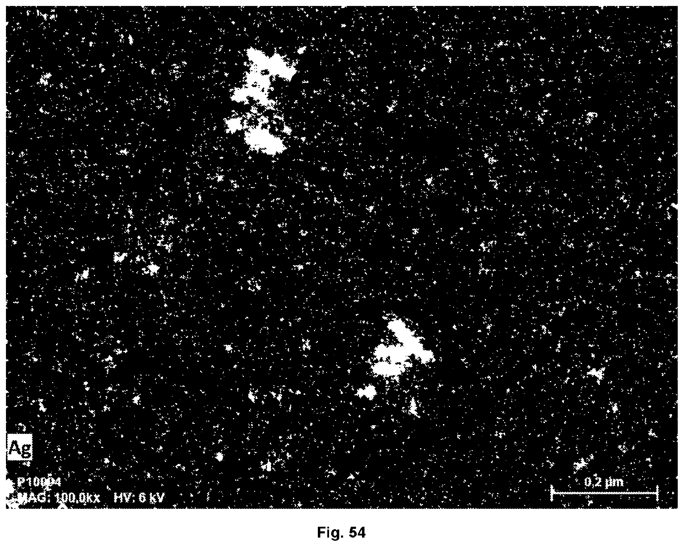

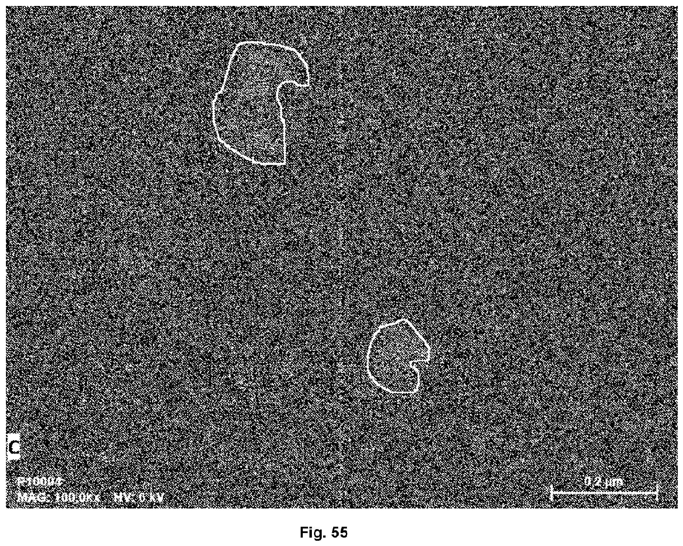

From Energy Dispersive X-Ray spectroscopy (Figures 44-58), it was possible to further verify the formation of silver nanoparticles. More interesting is the discovery of a biopolymer in solution, as highlighted from figures 56 to 58. It is possible to notice that carbon and oxygen atoms are present intra and inter-particle. This suggests the presence of carbohydrates capping the nanoparticles in solutions avoiding their agglomeration via steric hindrance and electrostatic repulsions. No other polymer or organic stabilising agents were added to the solution, meaning that these molecules originate from the original blends. This has the advantage of saving materials and production costs from the process since no extra stabiliser is required using our blends.

Finally, from tables 5 and 6, it is possible to notice several key advantages from the process of the present invention compared to other chemical reduction routes. Firstly, the use of the blends disclosed herein which have been optimised in cost, considerable cost savings can be made on the reducing agents as outlined on table 6. Moreover, the blends are non-toxic to humans nor present flammability or reactivity hazards making the product for the end user inherently safer compared to current market alternatives. Another key advantage is that it is possible to produce up to 5 times more the commercial concentration by using the process of the present invention in less than 30 minutes.

Table 5

| Temperature | Invention Concentration (M) | Market Concentration (M) | Invention: Market |
|---|---|---|---|
| 50°C | 3.73E-10 | 9.47E-11 | 3.9 |
| 60°C | 3.64E-10 | 9.47E-11 | 3.8 |
| 70°C | 5.27E-10 | 9.47E-11 | 5.6 |

Table 6

| Reducing Agents | Cost (£/g Dry) | Health (NFPA 704) | Flammability (NFPA 704) | Reactivity (NFPA 704) |
|---|---|---|---|---|
| Blend 1 | 0.16 | 0 | 0 | 0 |
| Blend 2 | 0.13 | 0 | 0 | 0 |
| Blend 3 | 0.19 | 0 | 0 | 0 |
| Blend 4 | 0.23 | 0 | 0 | 0 |
| Blend 5 | 0.30 | 0 | 0 | 0 |
| NaBH$_4$ | 1.53 | 3 | 1 | 2 |
| N,N-dimethylformamide | 0.34 | 2 | 2 | 0 |
| Hydrazine | 1.35 | 4 | 4 | 3 |

It can also be noted on the UV-Vis spectra that absorbances are detectable from t=0mins. Therefore, it can be deduced that the reaction is quasi-instantaneous upon contact of the reagents, hence opening the potential to continuous flow crystallisation opposed to batch flow, which is the current industry standard. This would present major scale-up advantages, making a previously known batch process into a continuous one. Also, we do not use any VOCs (Volatile Organic Compounds) or organics in our process either as solvents or for liquid-liquid extraction.

The process of the present invention is a greener and safer biochemical process alternative to current market products, from inorganic reduction routes to as well as the current plant-based reducing agent routes in the prior art.

**Comparative Example 1**

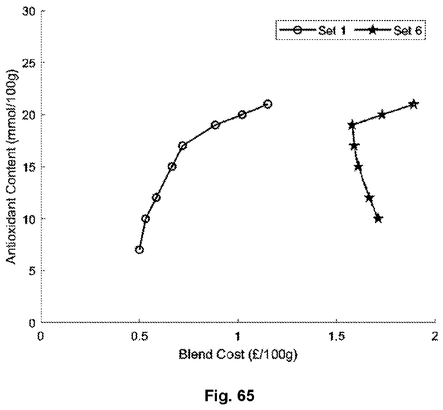

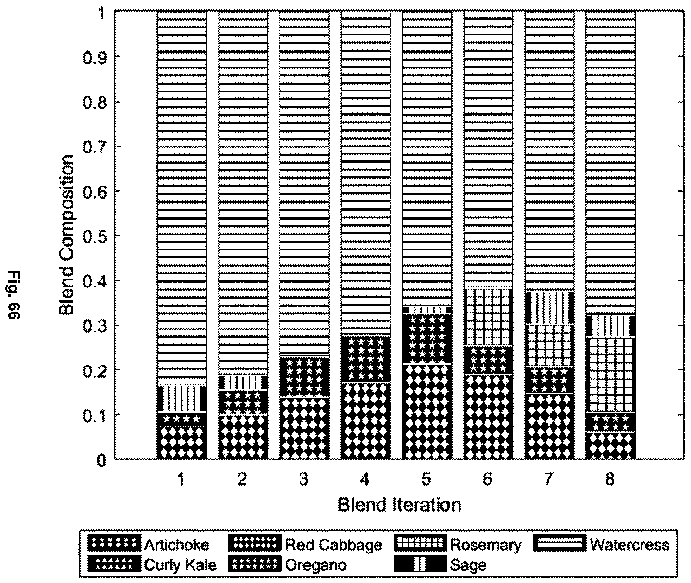

The possibility of replacing curly kale with another plant was evaluated. Since watercress has similar nutritional characteristics to curly kale it has been chosen as a possible replacement to it. As it is possible to see on Figures 65 and 66, watercress-based blends use sage, oregano and rosemary as high-quality antioxidant carriers and red cabbage as a filler. Nevertheless, it is possible to observe that to obtain the same results (in terms of antioxidant activity) as shown the cost would be increased between 30% to 70% compared to the curly kale model (see Table 7). This further validates the choice of curly kale over watercress as compared to current available literature findings.

Table 7

| Blend Identifier (BX) | Antioxidants (mmol/100g) | Cost (£/100g) - Curly Kale Blend | Cost (£/100g) - Watercress Blend | Cost Increase (%) |
|---|---|---|---|---|
| B1 | 7 | 0.501 | Infeasible | N/a |
| B2 | 10 | 0.532 | 1.71 | 69% |
| B3 | 12 | 0.586 | 1.664 | 65% |
| B4 | 15 | 0.666 | 1.61 | 59% |
| B5 | 17 | 0.719 | 1.59 | 55% |
| B6 | 19 | 0.886 | 1.58 | 44% |
| B7 | 20 | 1.02 | 1.73 | 41% |
| B8 | 21 | 1.15 | 1.89 | 39% |
| B9 | 22 | 1.486 | 2.09 | 29% |

**Example 5 - further method of producing a silver metal nanoparticle**

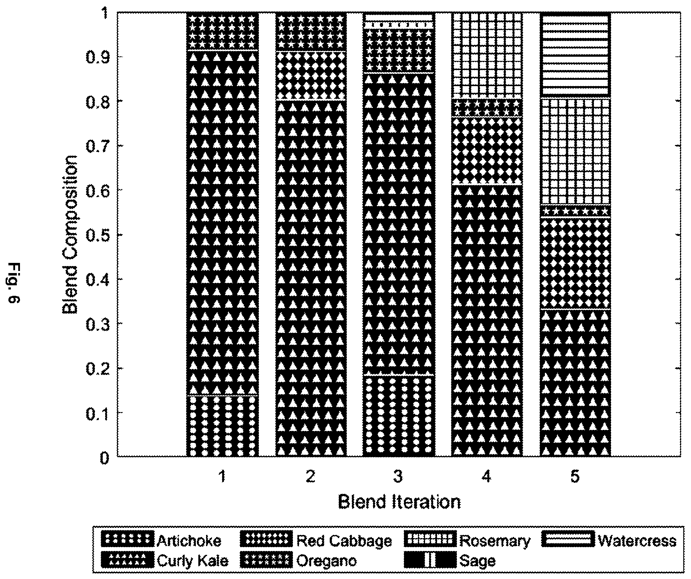

The method generally described in Example 4 was repeated to produce silver nanoparticles in four batches. The precise conditions are referred to in Table 8 below as S1 to S4, the concentrations varying between 0.25 g/L to 0.9 g/L. The stirring was carried out at 600 RPM. Any of blends 1 to 5 having the compositions illustrated in Fig. 6 are suitable for carrying out this method.

Following reaction at 70°C and under 600RPM stirring, the S1, S2 and S3 reaction mixture were quenched with ice cooled water after 5 minutes, and the S4 reaction mixture after 30 minutes. It was noted that the reaction was extremely fast - conversion of the silver ions to silver nanoparticles was observed for reaction mixtures S1, S2, S3 in less than 5 minutes.

Following recovery of the pellet, it was then ultrasonicated at 40kHz at 25°C first for 10 minutes, then shaken for 5 minutes, and then ultrasonicated at 40kHz at 25°C for another 5 minutes before being shaken again for 5 minutes.

Table 8

| Batch Name | Silver Acetate Concentration (g/L) | Blend Concentration (g/L) |
|---|---|---|
| S1 | 0.9 | 7.00E+00 |
| S2 | 0.7 | 7.00E+00 |
| S3 | 0.5 | 7.00E+00 |
| S4 | 0.25 | 7.00E+00 |

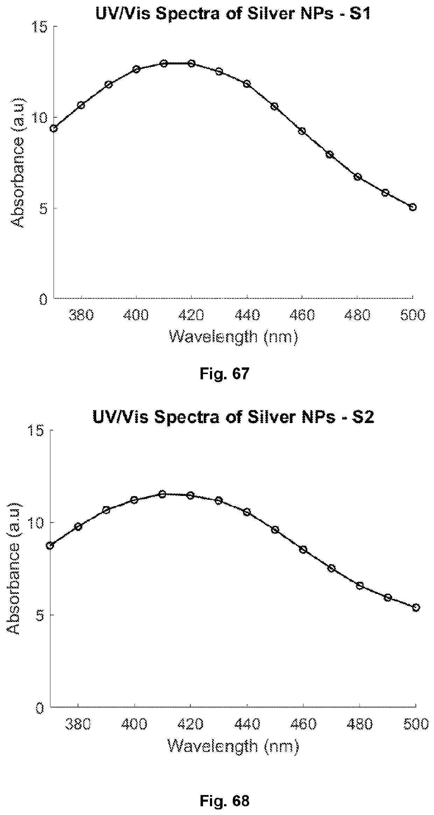

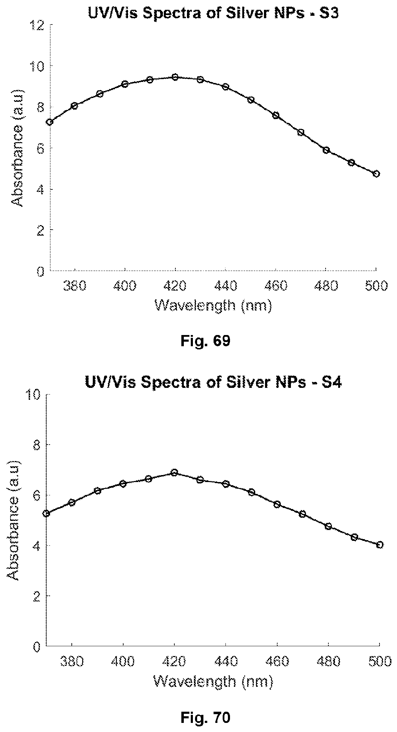

The diameter of the nanoparticles were estimated by UV-VIS spectroscopy using the same method detailed in Example 4. The UV-VIS spectra are shown in Figs. 67 to 70. The molar concentrations of the nanoparticles were also measured and compared with values for commercially available nanoparticles. The estimated diameters and concentrations are shown in Table 9.

Table 9

| Batch Name | Estimated Diameter (nm) | Invention Molar Concentration (mol/L) | Commercial Molar Concentration | Ratio (Invention: Commercial) |
|---|---|---|---|---|
| S1 | 38 | 4.39E-10 | 9.47E-11 | 4.64 |
| S2 | 38 | 3.91E-10 | 9.47E-11 | 4.12 |
| S3 | 50 | 1.76E-10 | 4.28E-11 | 4.11 |
| S4 | 50 | 1.28E-10 | 4.28E-11 | 2.99 |

This Example shows that silver nanoparticles having over 2.9 times, and in some cases over 4.5 times the concentration available on the market can be manufactured using the process of the present invention.

**Example 6 - general method of producing a gold metal nanoparticle**

An ionic solution was prepared at a desired concentration between 0.1 g/L to 1.5 g/L and is preheated in a reactor to 70°C for 1hr at 1 bar under continuous stirring at 600 RPM. When a steady temperature value is achieved, a blend concentration between 0.5 g/L to 10 g/L was added to the reactor and nucleation instantaneously initiates. Any of blends 1 to 5 having the compositions illustrated in Fig. 6 are suitable for this purpose. Gold (III) chloride hydrate ($HAuCl_4.H_2O$) was used.

The crystallisation lasted between 5 minutes to 2 hours depending on the desired conversion and nanoparticle size. It can be noticed that nanoparticles grow as more time lapses, therefore suggesting the tuning of nanoparticle size with reaction time. This is when the bio-reduction occurs and the metallic nanoparticles grow through primary heterogeneous nucleation. A colour change from transparent to winered/violet can be observed during this period.

The reaction was then quenched using cooling water at 4°C until the internal temperature of the mixture reaches 10°C. The mixture of nanoparticles and plant extract was subsequently recovered and centrifuged (10,000 rpm for 20 minutes). The supernatant was recycled back to the reactor in order to increase conversion. It passes through a cation exchange resin in order to recover a maximum amount of pure metal ion to be recycled back. Inactivated antioxidants in the process are purged. The pellet (with traces of water) was recovered and NaOH is added to increase the pH between 10 to 14. The pellet was then ultrasonicated at 40kHz at 25°C first for 10 minutes, then shaken for 5 minutes, and then ultrasonicated at 40kHz at 25°C for another 5 minutes before being shaken again for 5 minutes.

Thereafter, the colloid was filtered through a micromembrane in order to remove any large agglomerates or deformed particles to ensure high quality functional nanoparticles with high surface area to volume ratios. This is an especially useful property for medical, pharmaceutical, antimicrobial applications, conductive inks, and catalysis. Prior to shipping, the solution pH is adjusted to neutral or slightly alkaline (between pH=7 to pH=9) using a weak organic acid such as acetic acid in order for the solution not to be corrosive for users.

Gold nanoparticles were finally obtained in a colloidal solution. Sufficient nanoparticles were produced in each case and were identified using spectral analysis (UV/VIS). From UV-Vis analysis, it was possible to determine by known methods (described in https://www.sigmaaldrich.com/GB/en/technical-documents/technical-article/materials-scien ce-and-engineering/biosensors-and-imaging/gold-nanoparticles) that the nanoparticles produced at the reactor exit had diameters between 40 - 60 nm.

Post - reactor, the nanoparticles were filtered using microfiltration. 200nm membranes were used in order to discard any large agglomerate which may have remained post ultrasonication.

**Example 7 - specific method of producing a gold metal nanoparticle**

The method generally described in Example 6 was carried out to produce gold nanoparticles in three batches. The precise conditions are referred to in Table 11 below as S1 to S3. Any of blends 1 to 5 having the compositions illustrated in Fig. 6 are suitable for carrying out this method.

The particles were made in three batches, S1 to S3, having the concentrations in Table 10 below:

Table 10

| Batch Name | Gold (III) chloride hydrate Concentration (g/L) | Blend Concentration (g/L) |
|---|---|---|
| S1 | 0.9 | 7.00E+00 |
| S2 | 0.7 | 7.00E+00 |
| S3 | 0.25 | 7.00E+00 |

Following reaction at 70°C and under 600RPM stirring, the S1 and S2 reaction mixture were quenched with ice cooled water after 3 minutes, and the S3 reaction mixture after 30 minutes. It was noted that the reaction was extremely fast - conversion of the gold ions to gold nanoparticles was observed for reaction mixtures S1 and S2 in less than 3 minutes.

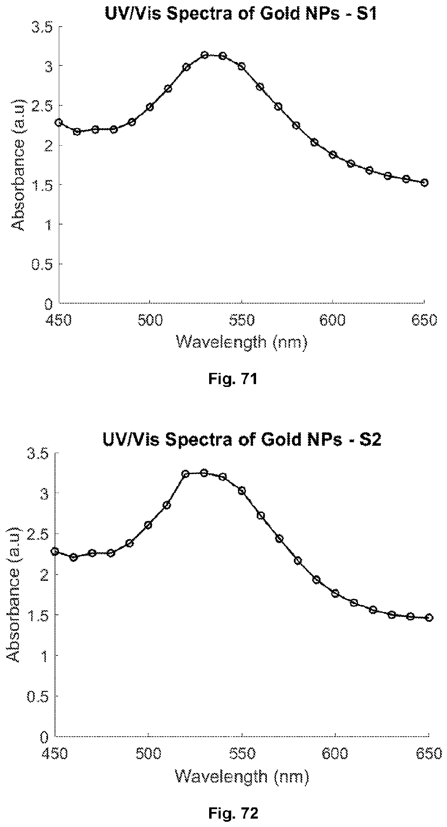

The diameter of the nanoparticles were estimated by UV-VIS spectroscopy using the same method detailed in Example 6. The UV-VIS spectra are shown in Figs. 71 to 73.

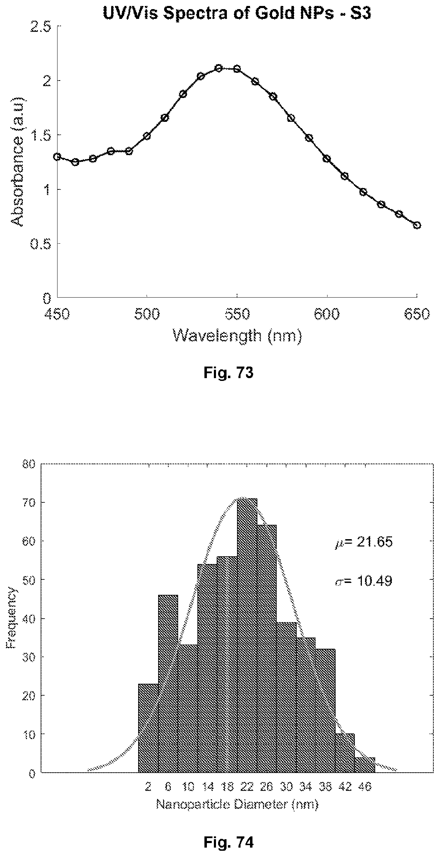

The particle size distribution of the gold nanoparticles is shown in Figure 74. From this figure, it is possible to observe that on a large sampling of nanoparticles a normal distribution is obtained which has an average diameter at 21.65 nm and a standard deviation of 10.49 nm.

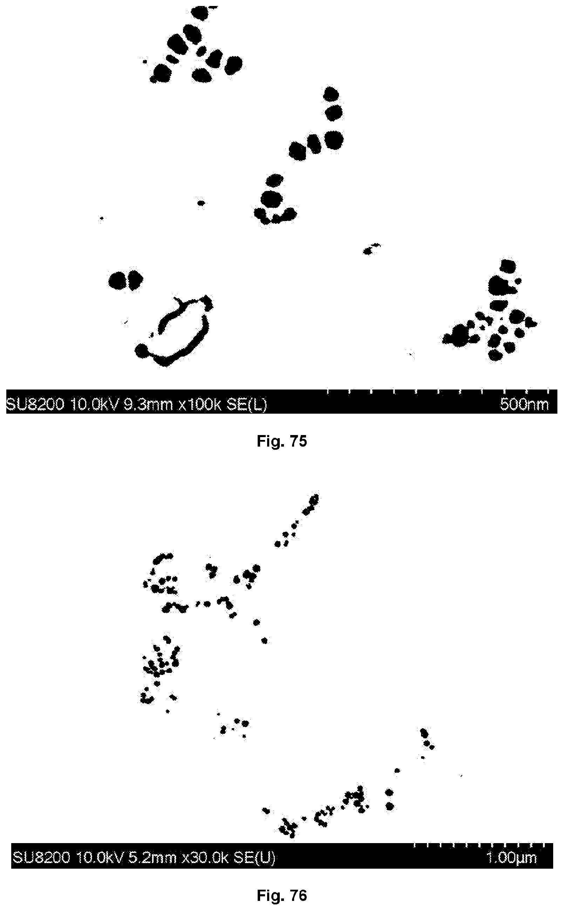

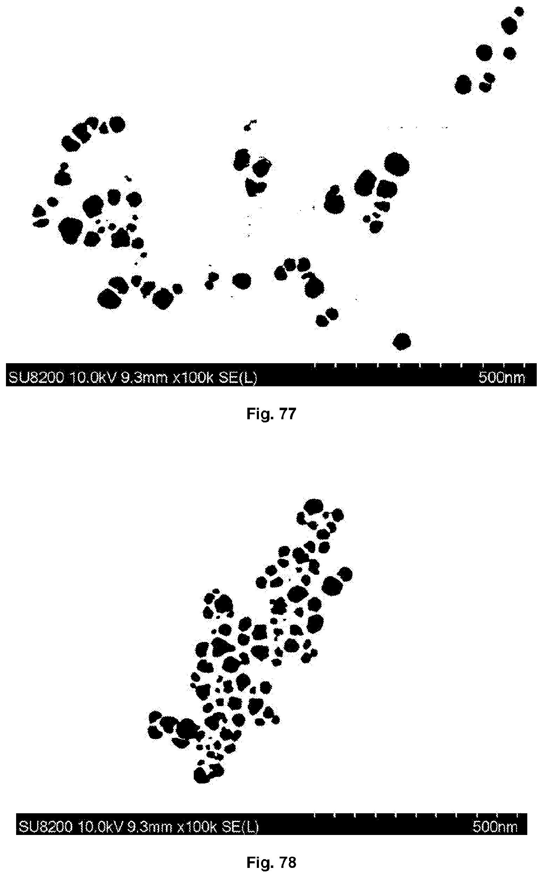

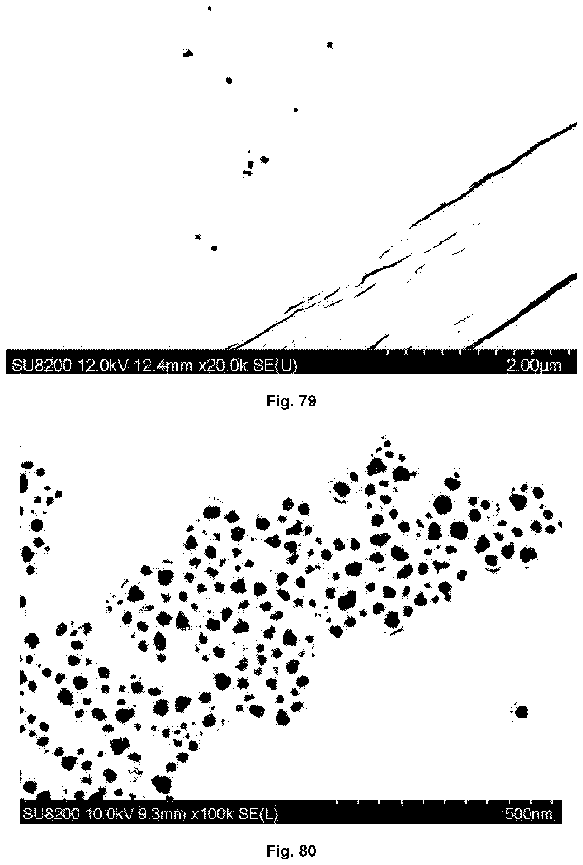

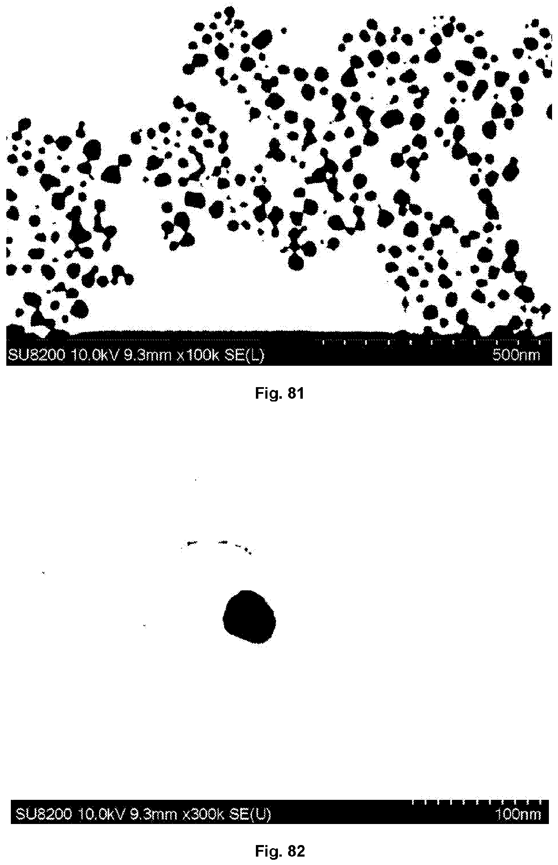

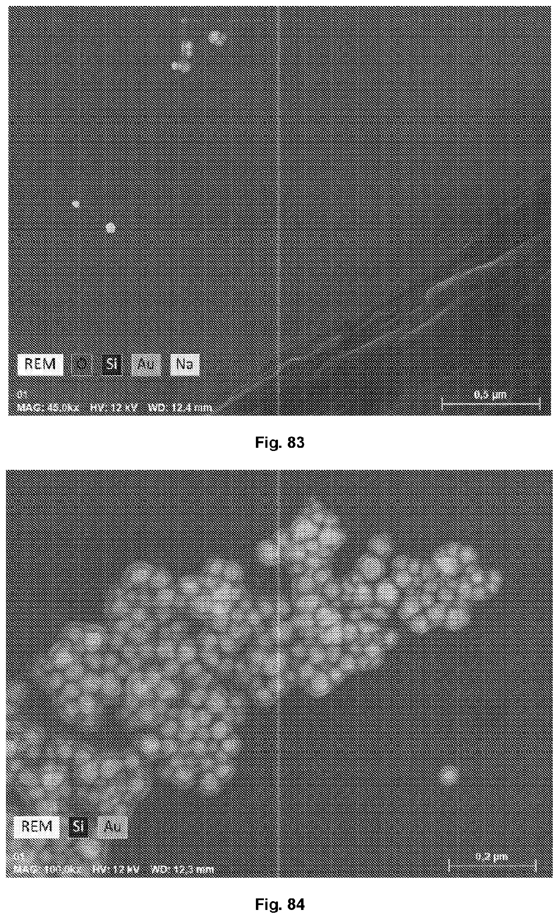

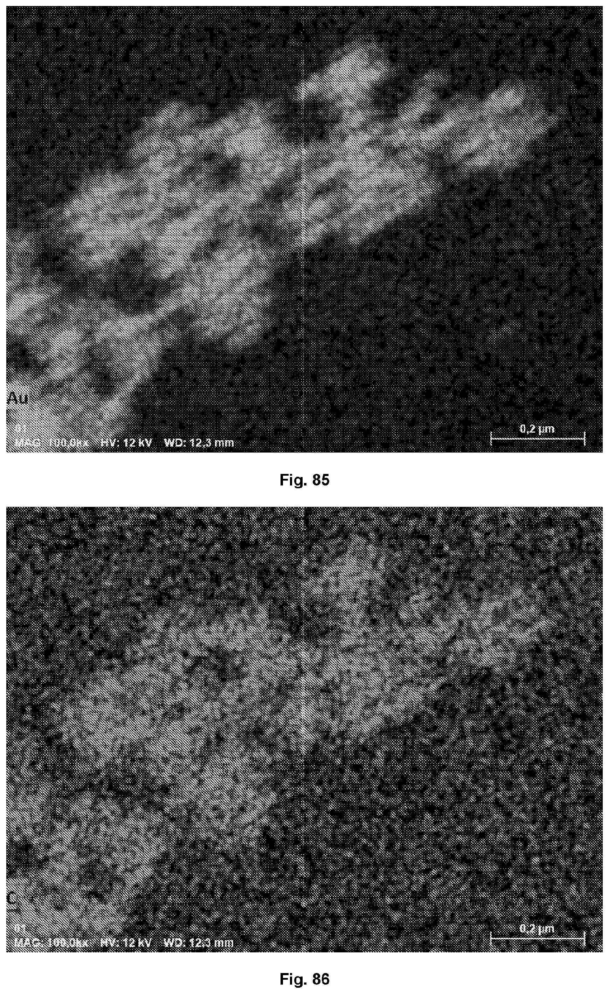

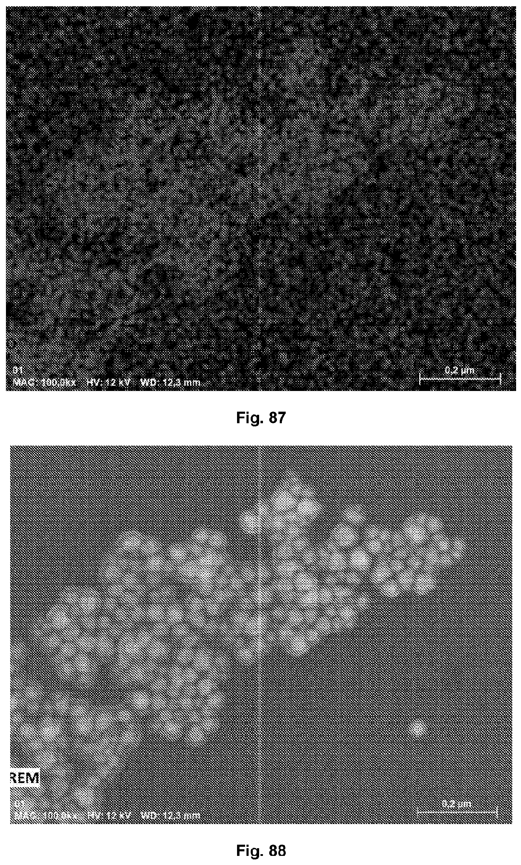

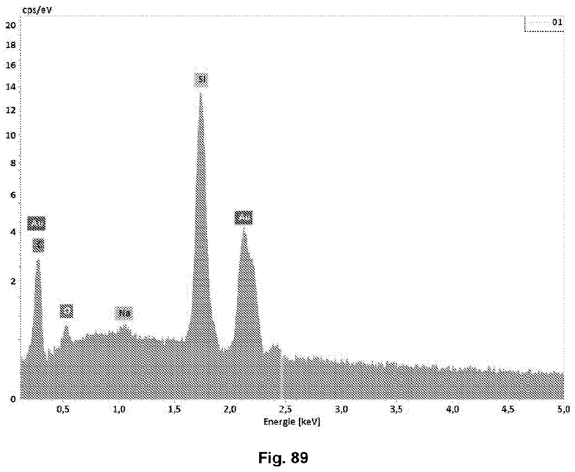

The gold nanoparticles were examined by scanning electron microscope) (SEM) as shown in Figures 75-82, and by Energy Dispersive X-Ray spectroscopy (EDX) as shown in Figures 83 and 84. From EDX it was possible to further verify the formation of gold nanoparticles, and also to superimpose the locations for the gold nanoparticles and carbon and oxygen atoms as shown in Figs. 85 to 88. This further confirms the presence of at least a carbohydrate bio-capping layer on the gold nanoparticles. Fig. 89 is an EDX Spectroscopy spectra of the gold nanoparticles, further supporting the presence of elemental gold as well as carbon and oxygen on the nanoparticles. This goes to further confirm the presence of a bio capping agent present on the metal nanoparticles.

The molar concentrations of the nanoparticles were also measured and compared with values for commercially available nanoparticles. The estimated diameters and concentrations are shown in Table 11.

Table 11

| Batch Name | Estimated Diameter (nm) | Invention Molar Concentration (mol/L) | Commercial Molar Concentration | Ratio (Invention: Commercial) |
|---|---|---|---|---|
| S1 | 40 | 3.72E-10 | 1.19E-10 | 3.13 |
| S2 | 40 | 3.86E-10 | 1.19E-10 | 3.24 |
| S3 | 60 | 6.87E-11 | 3.25E-11 | 2.11 |

This Example shows that gold nanoparticles having over 3 times the concentration available on the market can be manufactured using the process of the present invention.

## Claims

1. A method of producing a metal nanoparticle having a diameter between 1nm and 1μm, the method comprising:

   (a) providing a dissolved metal ion; and
   (b) contacting the dissolved metal ion with a composition comprising extracts from:

      (a) kale (*Brassica oleracea* Acephala group) and oregano (*Origanum vulgare*); and
      (b) at least one other plant selected from the group consisting of:

         (i) artichoke (*Cynara cardunculus*);
         (ii) red cabbage *(Brassica oleracea var.* Capitata group *var. rubra*);
         (iii) rosemary (*Salvia rosmarinus*);
         (iv) sage (*Salvia officinalis*);
         (v) watercress (*Nasturtium officinale*);

   such that the dissolved metal ion is reduced to form the metal nanoparticle.

2. A method according to claim 1, wherein the metal is selected from the group consisting of gold, silver, copper, nickel, platinum, iron, zinc, gadolinium, titanium, cerium, thallium, manganese, aluminium, zirconium, chromium, palladium and cobalt, or any combination thereof.

3. A method according to claim 1, wherein the metal is silver.

4. A method according to claim 1, wherein the metal is gold.

5. A method according to any one of claims 1 to 4, wherein the dissolved metal ion is present in the form of a solution of a metal salt.

6. A method according to claim 5, wherein the metal salt is an acetate salt.

7. A method according to any one of claims 1 to 6, wherein the composition comprises extracts from:

   (a) kale (*Brassica oleracea* Acephala group) and oregano (*Origanum vulgare*); and
   (b) at least one other plant selected from the group consisting of:

(i) artichoke (*Cynara cardunculus*);
(ii) red cabbage (*Brassica oleracea var.* Capitata group *var. rubra*);
(iii) rosemary (*Salvia rosmarinus*); and
(iv) watercress (*Nasturtium officinale*).

8.  A method according to any one of claims 1 to 6, wherein the composition comprises extracts from:

    (a) kale (*Brassica oleracea var.* Acephala group) and oregano (*Origanum vulgare*); and
    (b) at least two other plants selected from the group consisting of:

    (i) artichoke (*Cynara cardunculus*);
    (ii) red cabbage (*Brassica oleracea var.* Capitata group *var. rubra*);
    (iii) rosemary (*Salvia rosmarinus*); and
    (iv) watercress (*Nasturtium officinale*).

9.  A method according to any one of claims 1 to 6, wherein the composition comprises extracts from the following:

    (i) kale (*Brassica oleracea var.* Acephala group), in an amount of 30% to 90% by mass, as a proportion of the total mass of the composition;
    (ii) artichoke (*Cynara cardunculus*), in an amount of 0% to 20% by mass, as a proportion of the total mass of the composition;
    (iii) red cabbage (*Brassica oleracea var.* Capitata group *var. rubra*), in an amount of 0% to 30% by mass, as a proportion of the total mass of the composition;
    (iv) oregano (*Origanum vulgare*) in an amount of 1% to 20% by mass, as a proportion of the total mass of the composition;
    (v) rosemary (*Salvia rosmarinus*), in an amount of 0% to 30% by mass, as a proportion of the total mass of the composition; and
    (vi) watercress (*Nasturtium officinale*), in an amount of 0% to 30% by mass, as a proportion of the total mass of the composition.

10. A method according to any one of claims 1 to 9, wherein a solution of metal ions is added to the composition.

11. A method according to claim 10, wherein the solution of metal ions is pre-heated before addition of the composition.

**Patentansprüche**

1.  Verfahren zur Herstellung eines Metallnanopartikels mit einem Durchmesser zwischen 1 nm und 1$\mu$m, wobei das Verfahren umfasst:

    (a) Bereitstellen eines gelösten Metallions; und
    (b) Inkontaktbringen des gelösten Metallions mit einer Zusammensetzung umfassend Extrakte aus:

    (a) Grünkohl (Brassica oleracea, Acephala-Gruppe) und Oregano (Origanum vulgare); und
    (b) wenigstens einer anderen Pflanze ausgewählt aus der Gruppe bestehend aus:

    (i) Artischocke (Cynara cardunculus);
    (ii) Rotkohl (Brassica oleracea var. Capitata-Gruppe var. Rubra);
    (iii) Rosmarin (Salvia rosmarinus);
    (iv) Salbei (Salvia officinalis);
    (v) Wasserkresse (Nasturtium officinale);

    so dass das gelöste Metallion reduziert wird, um das Metallnanopartikel zu bilden.

2.  Verfahren nach Anspruch 1, wobei das Metall ausgewählt ist aus der Gruppe bestehend aus Gold, Silber, Kupfer, Nickel, Platin, Eisen, Zink, Gadolinium, Titan, Cer, Thallium, Mangan, Aluminium, Zirkonium, Chrom, Palladium und Cobalt oder einer beliebigen Kombination davon.

**3.** Verfahren nach Anspruch 1, wobei das Metall Silber ist.

**4.** Verfahren nach Anspruch 1, wobei das Metall Gold ist.

**5.** Verfahren nach einem der Ansprüche 1 bis 4, wobei das gelöste Metallion in der Form einer Lösung eines Metallsalzes vorliegt.

**6.** Verfahren nach Anspruch 5, wobei das Metallsalz ein Acetatsalz ist.

**7.** Verfahren nach einem der Ansprüche 1 bis 6, wobei die Zusammensetzung Extrakte umfasst aus:

(a) Grünkohl (Brassica oleracea, Acephala-Gruppe) und Oregano (Origanum vulgare); und
(b) wenigstens einer anderen Pflanze ausgewählt aus der Gruppe bestehend aus:

(i) Artischocke (Cynara cardunculus);
(ii) Rotkohl (Brassica oleracea var. Capitata-Gruppe var. Rubra);
(iii) Rosmarin (Salvia rosmarinus); und
(iv) Wasserkresse (Nasturtium officinale).

**8.** Verfahren nach einem der Ansprüche 1 bis 6, wobei die Zusammensetzung Extrakte umfasst aus:

(a) Grünkohl (Brassica oleracea var. Acephala-Gruppe) und Oregano (Origanum vulgare); und
(b) wenigstens zwei anderen Pflanzen ausgewählt aus der Gruppe bestehend aus:

(i) Artischocke (Cynara cardunculus);
(ii) Rotkohl (Brassica oleracea var. Capitata-Gruppe var. Rubra);
(iii) Rosmarin (Salvia rosmarinus); und
(iv) Wasserkresse (Nasturtium officinale).

**9.** Verfahren nach einem der Ansprüche 1 bis 6, wobei die Zusammensetzung Extrakte umfasst aus den Folgenden:

(i) Grünkohl (Brassica oleracea var. Acephala-Gruppe) in einer Menge von 30 bis 90 Masse-% als Anteil an der Gesamtmasse der Zusammensetzung;
(ii) Artischocke (Cynara cardunculus) in einer Menge von 0 bis 20 Masse-% als Anteil der Gesamtmasse der Zusammensetzung;
(iii) Rotkohl (Brassica oleracea var. Capitata-Gruppe var. Rubra) in einer Menge von 0 bis 30 Masse-% als Anteil an der Gesamtmasse der Zusammensetzung;
(iv) Oregano (Origanum vulgare) in einer Menge von 1 bis 20 Masse-% als Anteil der Gesamtmasse der Zusammensetzung;
(v) Rosmarin (Salvia rosmarinus) in einer Menge von 0 bis 30 Massen-% als Anteil an der Gesamtmasse der Zusammensetzung; und
(vi) Wasserkresse (Nasturtium officinale) in einer Menge von 0 bis 30 Massen-% als Anteil der Gesamtmasse der Zusammensetzung.

**10.** Verfahren nach einem der Ansprüche 1 bis 9, wobei eine Lösung von Metallionen zu der Zusammensetzung zugegeben wird.

**11.** Verfahren nach Anspruch 10, wobei die Lösung von Metallionen vor Zugeben der Zusammensetzung vorgewärmt wird.

**Revendications**

**1.** Procédé de production d'une nanoparticule métallique ayant un diamètre compris entre 1 nm et 1 µm, le procédé comprenant :

(a) la fourniture d'un ion métallique dissous ; et
(b) la mise en contact de l'ion métallique dissous avec une composition comprenant des extraits de :

(a) chou frisé (*Brassica oleracea var. acephala*) et origan (*Origanum vulgare*) ; et
(b) au moins une autre plante choisie dans le groupe constitué par :

(i) l'artichaut (*Cynara cardunculus*) ;
(ii) le chou rouge (*Brassica oleracea var.capitata var. rubra*) ;
(iii) le romarin (*Salvia rosmarinus*) ;
(iv) la sauge (*Salvia officinalis*) ;
(v) le cresson d'eau (*Nasturtium officinale*) ;

de telle sorte que l'ion métallique dissous est réduit pour former la nanoparticule métallique.

2.  Procédé selon la revendication 1, dans lequel le métal est choisi dans le groupe constitué par l'or, l'argent, le cuivre, le nickel, le platine, le fer, le zinc, le gadolinium, le titane, le cérium, le thallium, le manganèse, l'aluminium, le zirconium, le chrome, le palladium et le cobalt, ou une combinaison quelconque de ceux-ci.

3.  Procédé selon la revendication 1, dans lequel le métal est l'argent.

4.  Procédé selon la revendication 1, dans lequel le métal est l'or.

5.  Procédé selon l'une quelconque des revendications 1 à 4, dans lequel l'ion métallique dissous est présent sous la forme d'une solution d'un sel métallique.

6.  Procédé selon la revendication 5, dans lequel le sel métallique est un sel d'acétate.

7.  Procédé selon l'une quelconque des revendications 1 à 6, dans lequel la composition comprend des extraits de :

(a) chou frisé (*Brassica oleracea var. acephala*) et origan (*Origanum vulgare*) ; et
(b) au moins une autre plante choisie dans le groupe constitué par :

(i) l'artichaut (*Cynara cardunculus*) ;
(ii) le chou rouge (*Brassica oleracea var.capitata var. rubra*) ;
(iii) le romarin (*Salvia rosmarinus*) ; et
(iv) le cresson d'eau *(Nasturtium officinale).*

8.  Procédé selon l'une quelconque des revendications 1 à 6, dans lequel la composition comprend des extraits de :

(a) chou frisé (*Brassica oleracea var. acephala*) et origan *(Origanum vulgare)* ; et
(b) au moins deux autres plantes choisies dans le groupe constitué par :

(i) l'artichaut (*Cynara cardunculus*) ;
(ii) le chou rouge (*Brassica oleracea var.capitata var. rubra*) ;
(iii) le romarin (*Salvia rosmarinus*) ; et
(iv) le cresson d'eau (*Nasturtium officinale*).

9.  Procédé selon l'une quelconque des revendications 1 à 6, dans lequel la composition comprend des extraits des plantes suivantes :

(i) le chou frisé (*Brassica oleracea var. acephala*), à raison de 30 % à 90 % en masse, par rapport à la masse totale de la composition.
(ii) l'artichaut (*Cynara cardunculus*)*,* à raison de 0 % à 20 % en masse, par rapport à la masse totale de la composition ;
(iii) le chou rouge (*Brassica oleracea var. capitata var. rubra*)*,* à raison de 0 % à 30 % en masse, par rapport à la masse totale de la composition ;
(iv) l'origan (*Origanum vulgare*) à raison de 1 % à 20% en masse, par rapport à la masse totale de la composition ;
(v) le romarin (*Salvia rosmarinus*)*,* à raison de 0 % à 30 % en masse, par rapport à la masse totale de la composition ; et
(vi) le cresson d'eau (Nasturtium officinale), à raison de 0 % à 30 % en masse, par rapport à la masse totale de la composition.

**10.** Procédé selon l'une quelconque des revendications 1 à 9, dans lequel une solution d'ions métalliques est ajoutée à la composition.

**11.** Procédé selon la revendication 10, dans lequel la solution d'ions métalliques est préchauffée avant l'ajout de la composition.

Fig. 1

Fig. 1A

Fig. 2

Fig. 3

Fig. 4

Fig. 5

Fig. 6

**Fig. 7**

**Fig. 8**

**Fig. 9**

**Fig. 10**

**Fig. 11**

Fig. 12

Fig. 13

y=15.77x-19.79

**Fig. 14**

y=10.85x-34.52

**Fig. 15**

**Fig. 16**

**Fig. 17**

Fig. 18

Fig. 19

Fig. 20

Fig. 21

Fig. 22

Fig. 23

Fig. 24

Fig. 25

Fig. 26

Fig. 27

SU8230 6 0kV 10 1mm x3 00k SE(U) 10 0µm

Fig. 28

SU8230 6.0kV 10.1mm x10.0k SE(U) 5.00µm

Fig. 29

Fig. 30

Fig. 31

Fig. 32

Fig. 33

Fig. 34

SU8230 6.0kV 2.6mm x3.00k SE(U)    10.0μm

Fig. 35

SU8230 6.0kV 2.6mm x10.0k SE(U)    5.00μm

Fig. 36

Fig. 37

**Fig. 38**

SU8230 6.0kV 2.6mm x300k SE(U)　　　　100nm

**Fig. 39**

SU8230 6.0kV 2.6mm x3.00k SE(U)　　　　10.0μm

SU8230 6.0kV 2.6mm x10.0k SE(U)                    5.00µm

**Fig. 40**

SU8230 6.0kV 2.6mm x30.0k SE(U)                    1.00µm

**Fig. 41**

SU8230 6.0kV 2.6mm x100k SE(U)      500nm

Fig. 42

SU8230 6.0kV 2.6mm x300k SE(U)      100nm

Fig. 43

Fig. 44

**Fig. 45**

Fig. 46

Fig. 47

**Fig. 48**

Ag

P10094
MAG: 3000x   HV: 6 kV

10 µm

Fig. 49

**Fig. 50**

**Fig. 51**

**Fig. 52**

Fig. 53

**Fig. 54**

Fig. 55

**Fig. 56**

Fig. 57

Fig. 58

SU8230 6.0kV 10.1mm x100k PDBSE(CP)                 500nm

Fig. 59

SU8230 6.0kV 10.1mm x100k PDBSE(CP)                 500nm

Fig. 60

**Fig. 61**

**Fig. 62**

Fig. 63

Increasing Time

Fig. 64

**Fig. 65**

Fig. 66

**UV/Vis Spectra of Silver NPs - S1**

Fig. 67

**UV/Vis Spectra of Silver NPs - S2**

Fig. 68

Fig. 69

Fig. 70

**Fig. 71**

**Fig. 72**

**UV/Vis Spectra of Gold NPs - S3**

**Fig. 73**

$\mu$= 21.65

$\sigma$= 10.49

**Fig. 74**

SU8200 10.0kV 9.3mm x100k SE(L)                500nm

**Fig. 75**

SU8200 10.0kV 5.2mm x30.0k SE(U)                1.00µm

**Fig. 76**

SU8200 10.0kV 9.3mm x100k SE(L)      500nm

**Fig. 77**

SU8200 10.0kV 9.3mm x100k SE(L)      500nm

**Fig. 78**

SU8200 12.0kV 12.4mm x20.0k SE(U)    2.00µm

Fig. 79

SU8200 10.0kV 9.3mm x100k SE(L)    500nm

Fig. 80

SU8200 10.0kV 9.3mm x100k SE(L)          500nm

Fig. 81

SU8200 10.0kV 9.3mm x300k SE(U)          100nm

Fig. 82

Fig. 83

Fig. 84

Fig. 85

Fig. 86

**Fig. 87**

**Fig. 88**

Fig. 89

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120055873 A
- US 20110110723 A

**Non-patent literature cited in the description**

- **DAVID L. JOHNSON et al.** *Micro & Nano Letters*, 2020, vol. 15 (2), 110-113
- **MOHAMMAD POURHASSAN-MOGHADDAM et al.** *Micro & Nano Letters*, 2014, vol. 9 (5), 345-350
- **SOSNOWSKA MALWINA et al.** *Annals of Warsaw University of Life Sciences - SGGW - Animal Science*, 2017, vol. 56 (1), 137-145
- **JASMINE JACOB et al.** *Materials Science and Engineering C*, 2012, vol. 32 (7), 1827-1834
- **DEMIRBAS AYSE et al.** *Materials Letters*, 2016, vol. 179, 20-23
- **HASSANI SADI MOHADESEH et al.** IET Nanobiotechnology. The Institution of Engineering and Technology, 2017, vol. 11, 300-309
- **WIOLETTA FLORKIEWICZ et al.** IET Nanobiotechnology. The Institution of Engineering and Technology, 2019, vol. 13, 726-735
- **AZIZIAN-SHERMEH OMID et al.** *Journal of Inorganic and Organometallic Polymers and Materials*, 2021, vol. 31 (1), 279-291
- **JEONG SU J et al.** *Journal of the Science of Food and Agriculture*, 2019, vol. 100 (3), 1056-1063
- **PARAMELLE et al.** *Analyst*, 2014, vol. 139, 4855-4861